(19) **Europäisches Patentamt / European Patent Office / Office européen des brevets**

(11) **EP 1 582 234 B1**

(12) **EUROPÄISCHE PATENTSCHRIFT**

(45) Veröffentlichungstag und Bekanntmachung des Hinweises auf die Patenterteilung:
**20.01.2010 Patentblatt 2010/03**

(51) Int Cl.:
**A61N 1/37** (2006.01)

(21) Anmeldenummer: **05090051.3**

(22) Anmeldetag: **03.03.2005**

(54) **Herzschrittmacher**

Pacemaker

Stimulateur cardiaque

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **01.04.2004 DE 102004017137**

(43) Veröffentlichungstag der Anmeldung:
**05.10.2005 Patentblatt 2005/40**

(73) Patentinhaber: **Biotronik CRM Patent AG**
**6340 Baar (CH)**

(72) Erfinder:
• **Schermeier, Olaf**
**14059 Berlin (DE)**

• **Worzewski, Wolf**
**14169 Berlin (DE)**

(74) Vertreter: **Lindner-Vogt, Karin L. et al**
**Biotronik SE & Co. KG**
**Corporate Intellectual Properties**
**Woermannkehre 1**
**12359 Berlin (DE)**

(56) Entgegenhaltungen:
| | |
|---|---|
| **EP-A- 0 705 620** | **EP-A- 0 826 391** |
| **US-A- 5 247 929** | **US-A- 5 735 881** |
| **US-A- 5 861 012** | **US-A1- 2004 030 358** |
| **US-A1- 2005 021 095** | **US-B1- 6 345 201** |
| **US-B1- 6 389 316** | **US-B1- 6 434 428** |
| **US-B1- 6 584 354** | |

**Beschreibung**

Die Erfindung betrifft einen Elektrostimulator, insbesondere einen solchen implantierbaren Elektrostimulator für das Herz wie einen atrialen Herzschrittmacher oder einen Zweikammer-Herzschrittmacher im allgemeinen. Unter einem Zweikammer-Herzschrittmacher werden hier solche Herzschrittmacher oder implantierbare Elektrostimulatoren wie Kardioverter oder Defibrillatoren verstanden, die ausgebildet sind, wenigstens zwei Kammern eines Herzens zu stimulieren, z.B. Atrium und einen oder zwei Ventrikel.

Der Elektrostimulator weist Elektrodenanschlüsse auf, die mit einer Stimulationseinheit zum Abgeben von Stimulationsimpulsen über die Elektrodenanschlüsse verbunden sind, sowie eine Stimulationssteuereinheit, die mit der Stimulationseinheit verbunden und ausgebildet ist, die Abgabe von Stimulationsimpulsen auszulösen und die Stärke der Stimulationsimpulse zu bestimmen und hierzu ein Stimulationssteuersignal an die Stimulationseinheit abzugeben. Außerdem weist der Herzschrittmacher eine Detektionseinheit auf, die mit der Stimulationssteuereinheit und wenigstens indirekt mit den Elektrodenanschlüssen verbunden und ausgebildet ist, an den Elektrodenanschlüssen anliegende elektrische Potentiale auszuwerten und intrinsische Ereignisse zu detektieren.

Intrinsische Ereignisse werden auch als natürliche oder spontane Ereignisse oder als Eigenaktion bezeichnet und kennzeichnen das selbständige Kontrahieren einer Herzkammer wie Atrium oder Ventrikel. Herzkammer in Sinne dieser Beschreibung kann somit sowohl ein Atrium (Vorhof) als auch ein Ventrikel sein.

Die Erfindung betrifft außerdem einen atrialen Capture-Detektor, der wenigstens einen Signal- oder Dateneingang für kardiale Ereignisse repräsentierende Signale oder Daten aufweist.

Es ist eine übliche Eigenschaft von Herzschrittmachern, dass diese ein Herz mittels elektrischer Stimulationsimpulse zu einer Kontraktion anregen können. Je nach Bauart, Einstellung oder Programmierung des Herzschrittmachers werden Stimulationspulse unbedingt zu einem mittels eines Zeitgebers vorgegebenen Zeitpunkt abgegeben - dies entspricht einen getriggerten Modus des Herzschrittmachers - oder bedingt, dass heißt nur dann, wenn keine natürliche Kontraktion des Herzens innerhalb eines vorgegeben Zeitfensters (welches üblicherweise als Escape-Intervall bezeichnet wird) erfolgt. Der letztgenannte Betriebsmodus des Herzschrittmachers wird als inhibierter Modus bezeichnet, weil dass Erfassen einer natürlichen Herzkontraktion (erkennbar als sogenanntes intrinsisches Ereignis im Elektrokardiogramm (EKG)) innerhalb des Escape-Intervalls zum Inhibieren der Abgabe eines Stimulationsimpulses führt.

Bezüglich der Escape-Intervalle wird beispielsweise zwischen atrialen und ventrikulären Escape-Intervallen unterschieden, und zwar je nach dem, ob es um die das Auslösen atrialen Stimulationsimpulse zur Abgabe an das Atrium eines Herzens geht, oder um das Auslösen von ventrikulären Stimulationsimpulsen, die an den Ventrikel abgegeben werden sollen. Insbesondere die Abgabe ventrikulärer Stimulationsimpulse wird bei sogenannten AV-sequentiellen Herzschrittmachern so gesteuert, dass ein ventrikuläres Escape-Intervall (auch als AV-Intervall bezeichnet) durch ein atriales Ereignis gestartet wird. Das ventrikuläre Escape- oder AV-Intervall hat eine Dauer die in etwa der Dauer einer natürlichen Reizleitung vom Atrium zum Ventrikel entspricht. Häufig wird das AV-Intervall etwas länger eingestellt, um zu verhindern, dass ein Stimulationsimpuls einer natürlichen Kontraktion zuvorkommt oder gar mit einer gleichzeitigen natürlichen Kontraktion zusammenfällt. Im letztgenannten Fall (Zusammentreffen von natürlicher und stimulierter Kontraktion) spricht man von Fusionsereignissen (engl. Fusion beats). Eine derartige Verlängerung des AV-Intervalls wird auch als Hysterese bezeichnet.

Neben einer natürlichen Überleitung von Reizen vom Atrium zum Ventrikel kann es auch zu einer als retrograd bezeichneten Reizleitung vom Ventrikel zum Atrium kommen, mit der Folge dass eine ventrikuläre Kontraktion per retrograder Reizleitung mit entsprechender Verzögerung eine atriale Kontraktion auslöst. In der Regel ist ein derartiges, auf retrograder Reizleitung beruhendes atriales Ereignis unter physiologischen Gesichtspunkten verfrüht und kann im Zusammenhang mit Herzschrittmachern zu einer Schrittmacher-induzierten Tachycardie (pacemaker mediated tachycardia, PMT) führen.

Ein weiteres Problem besteht darin, dass das Fernfeld eines ventrikulären Stimulus auch im Atrium eines Herzens wahrzunehmen (zu erfassen) ist. Dies kann dazu führen, dass die Fernfeldwahrnehmung eines ventrikulären Stimulationsimpulses im Atrium fälschlich als intrinsisches, d.h. mit einer natürlichen Kontraktion einhergehendes atriales Ereignis gewertet wird. Diesem Problem wird üblicherweise mit einer postventrikulären, atrialen Refraktärzeit begegnet, die mit dem Auslösen eines ventrikulären Stimulus beginnt und innerhalb derer keine atrialen Ereignisse erfasst oder, wenn zwar erfasst, dann wenigstens nicht ausgewertet werden.

Elektrostimulatoren wie insbesondere Herzschrittmacher bewirken durch Abgabe eines ausreichend starken Stimulationsimpulses eine Muskelkontraktion. Bei implantierbaren Herzschrittmachern ist es grundsätzlich wünschenswert, wenn der jeweilige Stimulationsimpuls gerade ausreichend stark ist, um Myokard in der Umgebung der Stimulationselektrode ausreichend zu erregen. Diese zur Kontraktion des Myokards führende Erregung breitet sich dann ausgehend vom Stimulationsort auf natürliche Weise aus und führt im Ergebnis zu einer vollständigen Kontraktion der jeweiligen Herzkammer, d.h. Vorhof (Atrium) oder Ventrikel. Diejenige Stimulationsimpulsamplitude, die gerade ausreicht um ein Ansprechen (Capture) des Myokards auf einen Stimulationsimpuls hervorzurufen, wird Reizschwelle genannt. Ein eine Myokard-Kontraktion bewirkender Stimulationsimpuls oberhalb der Reizschwelle wird als überschwellig bezeichnet, ein keine Myokard-Kontraktion bewirkender Stimulationsimpuls unterhalb der Reizschwelle als unterschwellig.

Stimulationsimpulse, die gerade eine aus Sicherheitsgründen über der Reizschwelle liegende Impulsstärke aufweisen, benötigen weniger Energie, als noch stärkere Stimulationsimpulse. In Anbetracht der von vornherein beschränkten Energiekapazität implantierter Herzschrittmacher ist es von jeher ein Ziel, die jeweilige Reizschwelle am Stimulationsort möglichst genau zu erfassen, um die Impulsstärke oder Amplitude eines Stimulationsimpulses möglichst genau an die jeweils aktuelle Reizschwelle anpassen zu können. Die Reizschwelle kann sich im Laufe der Zeit verändern, daher ist ein einmaliger Reizschwellentest bei Implantation in der Regel nicht ausreichend.

Daher ist es bekannt, Herzschrittmacher mit Mitteln zur Stimulationserfolgskontrolle zu versehen, mit denen sich ermitteln lässt, ob ein abgegebener Stimulationsimpuls zum Ansprechen des Myokards geführt und eine entsprechende Reizantwort hervorgerufen hat.

Die (direkte) automatische Bestimmung des (Nicht-)Vorhandenseins einer Reizantwort nach einem Stimulationsimpuls durch die Messung des evozierten Potentials mit der gleichen Elektrode, mit der Stimulationsimpuls abgegeben wurde, dessen Wirksamkeit geprüft werden soll, ist für ventrikuläre Stimulationsimpulse bereits realisiert. Die entsprechenden Lösungsprinzipien können im Prinzip auch für die Vorhofebene gelten.

Tatsächlich gibt es aber für den Vorhofbereich z.Z. kein implantierbares oder externes System, mit dem ein vollautomatisches atriales "threshold-tracking" oder eine vollautomatische atriale Reizschwellenbestimmung zuverlässig durchgeführt werden kann.

Die fehlende Verfügbarkeit eines entsprechenden Systems für die atriale Applikation verwundert nicht, weil die evozierten Potentiale im Vorhof wegen dessen (im Vergleich zum Ventrikel) wesentlich geringeren Muskelmasse und wegen der Unterschiede im spezifischen Reizleitungssystem eine für die Wahrnehmung wesentlich schlechtere Signalqualität haben als die evozierten Ventrikelpotentiale.

Ansätze für das Schaffen eines atrialen Reizantwort- oder Capture Detektors sind aus den US 5,601,615, US 5,861,012 und US 6,584,354 bekannt. Diese bekannten Ansätze sind aus verschiedenen Gründen unbefriedigend.

Dokument US 2004/0030358 offenbart einen Elektrostimulator gemäß dem Oberbegriff des Anspruchs 1.

Es ist daher die der Erfindung zugrundeliegende Aufgabe, einen Herzschrittmacher anzugeben, der eine Stimulationserfolgskontrolle bietet, welche auch für das Atrium eines Herzens tauglich ist und Nachteile des Standes der Technik möglichst vermeidet.

Diese Aufgabe wird durch einen Elektrostimulator mit den Merkmalen des Patentanspruchs 1 gelöst. Vorteilhafte Ausführungsformen dieser Vorrichtung sind in den Unteransprüchen 2 bis 28 beschrieben.

Vorzugsweise ist die Stimulationssteuereinheit dazu ausgebildet, auf Basis einer vor dem vorgegebenen, mehrere Herzzyklen einschließenden Zeitraum anliegenden Stimulationsrate die Stimulationsrate während des vorgegebenen, mehrere Herzzyklen einschließenden Zeitraums so einzustellen, dass die eingestellte Stimulationsrate größer ist, als die zuvor anliegende Stimulationsrate. Während des vorgegebenen, mehrere Herzzyklen einschließenden Zeitraums erfolgt also eine Überstimulation, und zwar vorzugsweise mit einer um etwa 10 ppm (Pulse pro Minute) gegenüber der zuvor anliegenden Rate erhöhten Stimulationsrate.

Hierzu ist es nicht notwendig, eine vor dem vorgegebenen, mehrere Herzzyklen einschließenden Zeitraum herrschende natürliche Herzrate zu ermitteln. Dies schließt nicht aus, dass die vor dem vorgegebenen, mehrere Herzzyklen einschließenden Zeitraum anliegende Stimulationsrate auf einer zusammen mit der Detektionseinheit ermittelten natürlichen Herzrate beruht.

Als eingestellte Stimulationsrate kann aber alternativ auch diejenige Stimulationsrate angewandt werden, die zu Beginn des vorgegebenen Zeitraums besteht, es muss also nicht unbedingt eine höhere Rate eingestellt werden, wenn beispielsweise die zuvor anliegende Rate bereits wegen körperlicher Aktivität des Patienten oder aus anderen Gründen erhöht ist.

Zu beachten ist, dass die hier vorgestellte Erfindung keine Messung des evozierten Potentials erfordert und deshalb zum einen den mit dieser Messung verbundenen technischen Aufwand und zum anderen bestimmte Nachteile vermeidet, die eine Methode hat, die darauf beruht, dass nur für kurze Zeit nach dem Impuls geprüft wird, ob ein evoziertes Potential vorhanden ist (US 5,601,615), bei der aber die Wechselwirkung zwischen der Stimulation und dem Eigenrhythmus (natürlicher Herzrhythmus) unberücksichtigt bleibt. Zu diesen Nachteilen gehören Unsicherheiten die bei den bisherigen Lösungsansätzen unter bestimmten Voraussetzungen eine eingeschränkte Sensitivität und/oder Spezifität des Algorithmus für die automatische Erkennung der atrialen Reizantwort zur Folgen haben können (z.B. im Falle einer atrialen Fusionssystole oder bei zu hoher Polarisationsspannung nach der Impulsabgabe).

Die vorgestellte Erfindung ist somit nicht auf die problematische Messung evozierter Signale nach den einzelnen Vorhofimpulsen (atriale Stimulationsimpulse) angewiesen, sondern beurteilt für die Wirksamkeitskontrolle (capture control) der Vorhofstimulation deren Auswirkungen auf den atrialen Eigenrhythmus (natürlicher Herzrhythmus) während eines bestimmten Zeitfensters (vorgegebener Zeitraum): Eine unwirksame Vorhofstimulation hat keinen Einfluss auf den Vorhofrhythmus und führt dazu, dass ein intrinsischer Vorhofrhythmus in Erscheinung tritt und vom Schrittmacher mit Hilfe von Markern für atriale Senseereignisse registriert werden kann. Demgegenüber wird der intrinsische Vorhofrhythmus (natürlicher Herzrhythmus oder Eigenrhythmus im Atrium) durch eine wirksame Vorhofstimulation vorzugsweise mit einer Frequenz (eingestellte Stimulationsrate), die größer ist als die atriale Eigenfrequenz (natürliche Herzrate) unterdrückt und kann nicht in Erscheinung treten, was durch ein Fehlen atrialer Senseereignisse (detektierte intrinsische atriale Ereignisse) festgestellt werden kann.

In Fällen, in denen Zweifel an der atrialen Impulsbeantwortung (atrial capture) bestehen, kann z.B. im DDD-Modus mit einer abgeleiteten oder eingestellten Stimulationsfrequenz stimuliert werden, die etwa 10 ppm (Pulse pro Minute) höher ist als eine zuvor z.B. während des atrialen Wahrnehmungstests festgestellte, atriale Eigenfrequenz.

Vorzugsweise sind ist der Elektrostimulator als atrialer Herzschrittmacher, die Stimulationseinheit zum Erzeugen und Abgeben atrialer Stimulationsimpulse, die Detektionseinheit zum Erfassen intrinsischer atrialer Ereignisse und Stimulationssteuereinheit zum Ermitteln eines natürlichen atrialen Herzrhythmus auf Basis von der Detektionseinheit erfasster intrinsischer Ereignisse im Atrium ausgebildet. Diese Ausführungsvariante trägt der Tatsache Rechnung, dass der hier vorgestellte Reizantwort Detektor grundsätzlich auch zum detektieren eines ventrikulären Stimulationserfolges geeignet ist, sich aber vor allem auch für die atriale Stimulationserfolgskontrolle eignet.

Vorzugsweise ist hierbei die Stimulationssteuereinheit ausgebildet, in dem vorgegebenen, mehrere Herzzyklen einschließenden Zeitraum atriale Stimulationsimpulse mit einer eingestellten Stimulationsrate in einem getriggerten Modus auszulösen, bei dem atriale Stimulationsimpulse nicht inhibiert werden, und ventrikuläre Stimulationsimpulse mit einer eingestellten Stimulationsrate in einem Demand- Modus auszulösen, bei dem ventrikuläre Stimulationsimpulse inhibiert werden, falls innerhalb eines jeweiligen ventrikulären Escape-Intervalls intrinsische ventrikuläre Ereignisse erfasst werden. Der besondere Vorteil dieses Elektrostimulators besteht darin, dass eine unbeeinträchtigte Stimulation des Ventrikels erfolgen kann, während gleichzeitig eine atriale Stimulationserfolgskontrolle, ein atrialer Reizschwellentest oder gar eine automatische Anpassung der atrialen Stimulationsimpulsstärke auf Basis der atrialen Stimulationserfolgskontrolle erfolgen kann.

Für die atriale Stimulationserfolgskontrolle, den atrialen Reizschwellentest oder die automatische Anpassung der atrialen Stimulationsimpulsstärke ist die Stimulationssteuereinheit vorzugsweise ausgebildet, das Vorliegen oder Nichtvorliegen eines natürlichen Herzrhythmus in Abhängigkeit davon zu ermitteln, ob und/oder wie viele intrinsische Ereignisse von der Detektionseinheit innerhalb des vorgegebenen Zeitraums detektiert werden. Dieses Detektionskriterium ist besonders einfach zu implementieren und bereits ausreichend aussagekräftig.

Außerdem ist die Stimulationssteuereinheit in einer bevorzugten Ausführungsvariante ausgebildet, für die Ermittlung des Vorliegens oder Nicht-Vorliegens eines natürlichen Herzrhythmus Ausblendzeiten (Blankingzeiten), in denen eventuell auftretende natürliche Ereignisse nicht erfasst werden können, zu berücksichtigen. Dies kann beispielsweise dadurch erfolgen, dass für die Ausblendzeit pauschal ein Bruchteil eines intrinsischen Ereignisses gezählt wird, und zwar entsprechend der Wahrscheinlichkeit, dass ein intrinsisches Ereignis bei Annahme eines regelmäßigen natürlichen Rhythmus in die Ausblendzeit fällt. Wird beispielsweise ein tatsächlich erfasstes intrinsisches Ereignis mit "1" gezählt, kann je nach Länge der Ausblendzeiten und Wert der angenommen natürlichen Rate für jede Ausblendzeit in dem vorgesehenen Zeitraum ein Wert von "0,3" gezählt werden.

In diesem Zusammenhang kann die Stimulationssteuereinheit dazu ausgebildet sein, anhand erfasster natürlicher Ereignisse unter Berücksichtigung potentiell in den Ausblendzeiten aufgetretener, nicht erfasster natürlicher Ereignisse einen natürlichen Herzrhythmus zu rekonstruieren und ein einen natürlichen Herzrhythmus kennzeichnendes Signal zu erzeugen, falls die erfassten natürlichen Herzereignisse einem regelmäßigen, natürlichen Herzrhythmus zuzuordnen sind.

Eine vorteilhafte, weil einfach zu realisierende Ausführungsvariante ergibt sich, wenn die Detektionseinheit ausgebildet ist für iedes detektierte intrinsischer Ereignis ein Sense-Markersignal in relativer zeitlicher Zuordnung mit bezug auf den Zeitpunkt des Auftretens des Ereignisses zu erzeugen. Die Stimulationssteuereinheit ist dann vorzugsweise dazu ausgebildet, für jeden tatsächlich ausgelösten Stimulationsimpuls jeweils ein Pace-Markersignal zu erzeugen und das Vorliegen eines natürlichen Herzrhythmus auf Basis wenigstens der Sense-Markersignale oder zusätzlich der Pace-Markersignale zu ermitteln.

Ein besonders bevorzugter Elektrostimulator mit einer automatischen Anpassung der Stimulationsimpulsstärke ergibt sich, wenn die Stimulationssteuereinheit ausgebildet ist, ein Stimulationssteuersignal für die Stimulationsstärke abhängig davon zu bestimmen, ob ein Vorliegen oder ein Nichtvorliegen eines natürlichen Herzrhythmus innerhalb des vorgegebenen Zeitraums ermittelt ist. Hierzu ist die Stimulationssteuereinheit vorzugsweise ausgebildet, ein Stimulationsteuersignal, welches eine Erhöhung der Impulsstärke eines Stimulationsimpulses bewirkt, dann zu erzeugen, wenn ein Vorliegen eines natürlichen Herzrhythmus innerhalb des vorgegebenen Zeitraums ermittelt ist.

Bereits ein Elektrostimulator, dessen Stimulationssteuereinheit ausgebildet ist, ein Capture-Control-Signal in Abhängigkeit davon zu bestimmen, ob ein Vorliegen oder ein Nichtvorliegen eines natürlichen Herzrhythmus innerhalb des vorgegebenen Zeitraums ermittelt ist und zusätzlich oder alternativ im Falle des Vorliegens eines natürlichen Herzrhythmus innerhalb des vorgegebenen Zeitraums ein Loss-of-Capture-Signal (LOC) zu erzeugen, bietet den Vorteil einer effektiven Stimulationserfolgskontrolle.

Die Stimulationssteuereinheit ist dazu ausgebildet, wenigstens für eine begrenzte, insbesondere der Stimulationserfolgskontrolle (Capture-Detektion) oder einem Reizschwellentest dienenden Zeitraum ventrikuläre Stimulationsimpulse mit Ablauf eines mit einem intrinsischen oder stimulierten atrialen Ereignis beginnenden AV-Intervalls auszulösen, welches derart kurz ist, dass Fusionsereignisse, d.h. Ereignisse bei denen ein ventrikulärer Stimulationsimpuls im wesentlichen zeitgleich mit einer natürlichen ventrikulären Kontraktion erfolgt, vermieden werden.

Weiterhin ist die Stimulationssteuereinheit ausgebildet, atriale Ereignisse, die auf Fernfeldwahrnehmung ventrikulärer Ereignisse im Atrium beruhen von intrinsischen atrialen Ereignissen zu unterscheiden, indem solche atrialen Ereignisse, die einen Zeitabstand zum jeweils vorangehenden ventrikulären Stimulationsimpuls haben, der dem zeitlichen Abstand zwischen einem ventrikulären Stimulationsimpuls und dem entsprechenden atrialen Ereignis in einem Beobachtungszeitraum, beispielsweise in einem vorangehenden Herzzyklusses wenigstens annähernd entspricht, als auf Fernfeldwahrnehmung basierend gewertet und nicht als intrinsische Ereignisse gezählt werden.

Hierzu ist die Stimulationssteuereinheit vorzugsweise dazu ausgebildet, ein auf Fernfeldwahrnehmung basierende atriale Ereignisse kennzeichnendes Farfield-Signal zu erzeugen, falls die Detektionseinheit ein intrinsisches atriales Ereignis innerhalb eines vorgegebenen Farfield-Koppelintervalls detektiert, das nach Auslösen eines ventrikulären Stimulationsimpulses beginnt. Das Farfield-Koppelintervall beginnt vorzugsweise 50 bis 100 ms nach Auslösen des ventrikulären Stimulationsimpulses und 150 bis 200 ms nach dem Auslösen desselben ventrikulären Stimulationsimpulses endet.

Diese Lösung erlaubt eine besonders kurze postventrikuläre atriale Refraktärzeit, so dass besonders wenige atriale Ereignisse deshalb nicht wahrgenommen werden, weil sie in die atriale Refraktärzeit fallen. Der dadurch gesteigerten Gefahr, solche atrialen Ereignisse wahrzunehmen und fälschlicherweise als intrinsische Ereignisse zu interpretieren, die auf der Wahrnehmung des Fernfeldes ventrikulärer Stimulationsimpulse im Atrium beruhen, wird dadurch begegnet, dass atriale Ereignisse, die einen typischen, für die Fernfeldwahrnehmung charakteristischen zeitlichen Abstand zum vorangegangenen ventrikulären Stimulus haben, zwar aufgenommen, aber nicht als intrinsische Ereignisse gewertet werden.

Bevorzugte Ausführungsvarianten der Erfindung betreffen die Behandlung von retrograder Reizleitung und Übersprechen (Crosstalk).

In Bezug auf solche vermeintlichen atrialen Ereignisse, die entweder auf einer retrograden Reizleitung von Ventrikel zum Atrium oder auf Übersprechen zwischen den Elektrodenleitungen (CrossTalk) ist der erfindungsgemäße Elektrostimulator vorzugsweise wie folgt ausgebildet: Die Stimulationssteuereinheit ist ausgebildet, ein Retrograde-Reizleitungs-Signal zu erzeugen, falls die Detektionseinheit ein intrinsisches atriales Ereignis innerhalb eines vorgegebenen zeitlichen Abstandes nach Auslösen einen ventrikulären Stimulationsimpulses detektiert. Der vorgegebene zeitliche Abstand ist dabei vorzugsweise durch ein Koppelintervall von 180 ms bis 350 ms zwischen Auslösen des ventrikulären Stimulationsimpulses und Detektieren des intrinsischen atrialen Ereignisses gegeben.

Alternativ oder zusätzlich ist die Stimulationssteuereinheit dazu ausgebildet, ein Loss-of-Capture-Signal (LOC) zu erzeugen, falls das Verhältnis der Anzahl von Retrograden-Reizleitungs-Signalen zur Anzahl ausgelöster ventrikulärer Stimulationsimpulse innerhalb eines vorgegebenen Zeitraums eine vorgegeben Schwellwert zwischen 0,5 und 1 überschreitet. Der vorgegebene Schwellwert beträgt vorzugsweise 0,8.

Alternativ oder zusätzlich ist die Stimulationssteuereinheit dazu ausgebildet, ein Crosstalk-Signal zu erzeugen, falls die Detektionseinheit ein intrinsisches atriales Ereignis innerhalb eines vorgegebenen Zeitfensters detektiert, das mit Auslösen eines jeweiligen ventrikulären Stimulationsimpulses beginnt. Das vorgegebene Zeitfenster hat vorzugsweise eine Dauer von 30 ms bis 70 ms, besonders bevorzugt 50 ms.

Ein besonders bevorzugter Elektrostimulator besitzt eine Detektionseinheit, die ausgebildet ist, nur solche atrialen Ereignisse als intrinsische Ereignisse zu detektieren, denen kein Crosstalk-Signal oder kein Fafield-Signal zuzuordnen ist.

Eine ergänzende Lösung des Problems der Stimulationserfolgskontrolle im Atrium bietet ein Elektrostimulator der Eingangs genannten Art, der eine Stimulationssteuereinheit aufweist, die zusätzlich oder alternativ zu den zuvor geschilderten Merkmalen ausgebildet ist zum Zwecke der Stimulationserfolgskontrolle (Capture-Detektion) im Atrium wenigstens einen atrialen Stimulationsimpuls auszulösen, und einen Stimulationserfolg (Capture) im Atrium oder eine unwirksame atriale Stimulation (Non-capture) anhand des Nicht-Detektierens beziehungsweise des Detektierens wenigstens eines gegebenenfalls auftretenden intrinsischen (natürlichen, spontanen) atrialen Ereignisses innerhalb eines vorgegebenen Zeitraums zu erfassen.

Das der vorliegenden Erfindung zugrundeliegende Verfahren kann auch angewandt werden, wenn nur ein Oberflächen-EKG für die Analyse zur Verfügung steht (ohne IEGM/Marker-Registrierung) und evozierte P-Wellen nicht eindeutig erkennbar sind. Die Unwirksamkeit der Vorhofstimulation wird dann daran erkannt, dass bei ansonsten AVsequentieller Stimulation (sog. AV-Sequenzen) in mehr oder weniger regelmäßigen Abständen Kammerimpulse registriert werden, die von atrialen Senseereignissen getriggert worden sein mussten (sog. PV-Sequenzen). Dabei kommt es zu PV-Sequenzen immer dann, wenn ein atriales Senseereignis außerhalb der atrialen Refraktärperiode (ARP) wahrgenommen wird. Die Analyse wird durch die gleichzeitige Registrierung eines Oberflächen-EKGs mit atrialen Sensemarkern innerhalb und außerhalb der ARP wesentlich erleichtert, weil damit alle spontanen Vorhofdepolarisationen erfasst werden, außer denjenigen, die in die atriale Blankingzeit, die von einem Vorhofimpuls (sog. inchannel blanking) oder von einem Kammerimpuls (sog. cross-channel blanking) gestartet wird, fallen und deshalb nicht wahrgenommen werden können.

Mit dieser Registrierung lässt sich in der Regel die Existenz eines atrialen Eigenrhythmus eindeutig nachweisen, der von der Vorhofstimulation nicht unterdrückt wird, die deshalb unwirksam sein muss.

Die Erfindung macht sich diese Zusammenhänge zunutze und stellt einen Algorithmus vor, der aus dem Fehlen atrialer Senseereignisse während eines (gegebenenfalls programmierbaren) Zeitfensters auf die Wirksamkeit der Vorhofstimulation schließt.

In einer bevorzugten Ausführungsvariante wird dabei der (automatische) atriale Wahrnehmungstest mit der (automatischen) Prüfung der atrialen Impulsbeantwortung kombiniert.

In einer ersten Phase wird ein atrialer Wahrnehmungstest durchgeführt, der vier Zwecken dient. Es soll festgestellt werden:

1. ob der Patient einen atrialen Eigenrhythmus hat,
2. wie hoch dessen Frequenz ist,
3. ob die programmierte Vorhofempfindlichkeit ausreicht, um die atrialen Eigenaktionen wahrzunehmen, und
4. ob bei der programmierten Vorhofempfindlichkeit und Dauer der atrialen Blankingzeit (Far Field Blanking) eine Vorhofwahrnehmung des QRS-Komplexes (sog. Fernfeld-R-Wahrnehmung, "cross-talk") stattfindet.

Im Anschluss an den atrialen Wahrnehmungstest kann in einer zweiten Phase eine Prüfung der atrialen Impulsbeantwortung erfolgen. Wenn bei dem atrialen Wahrnehmungstest, der z.B. im VDD-Modus mit einer niedrigen Grundfrequenz von z.B. 40 ppm und mit kurzer AV-Zeit von z.B. 100 ms durchgeführt wird, eine regelmäßige atriale Eigenfrequenz zwischen 40/min und 100/min gemessen wird, erfolgt die Testphase zur Prüfung der atrialen Impulsbeantwortung. In einer bevorzugten Anwendung wird für diese Testphase der DDD-Modus mit einer atrialen Refraktärzeit von 775 ms (damit atriale Senseereignisse nicht außerhalb der atrialen Refraktärzeit einfallen und eine AV-Zeit starten können), einer Grundfrequenz, die etwa 20% höher ist als die zuvor gemessene atriale Eigenfrequenz, und einer AV-Zeit von 150 ms (die nicht länger sein sollte, damit es bei Blockierung im proximalen AV-Knoten nicht zu einer VA-Leitung kommen kann). In dieser zweiten Testphase wird nach vier Stimulationszyklen das Zeitfenster (bestehend aus n, mindestens 8, Zyklen) für die Messung der Intervalle zwischen den atrialen Senseereignissen gestartet und die resultierenden Ereignisse wie folgt ausgewertet:

- Wenn kein oder nur ein atriales Senseereignis registriert wird, gilt die Vorhofstimulation als wirksam;

- wenn mindestens drei Intervalle zwischen den atrialen Senseereignissen mit den beim atrialen Wahrnehmungstest gemessenen (plus/minus Toleranz von 50ms - 100 ms, die idealerweise frequenzabhängig ist) übereinstimmen, gilt die Vorhofstimulation als unwirksam und es wird davon ausgegangen, dass keine VA-Leitung nach der unwirksamen Vorhofstimulation stattfand.

- Wenn regelmäßige atriale Senseereignisse mit einer festen Kopplung im Bereich von ca. 180ms - 300 ms nach dem ventrikulären Stimulus auftreten, gilt die Vorhofstimulation ebenfalls als unwirksam und es wird davon ausgegangen, dass eine VA-Leitung nach der unwirksamen Vorhofstimulation stattgefunden hat.

- Ansonsten (d.h. bei zwei oder mehr atrialen Senseereignissen, deren Intervalle nicht die oben erwähnten Bedingungen erfüllen) wird davon ausgegangen, dass keine eindeutige Aussage über die (Un-)Wirksamkeit der Vorhofstimulation getroffen werden kann; der Anwender muss dann die Dokumentation des Tests analysieren, um die Ursache für das nicht eindeutige Testergebnis herauszufinden. Die fehlende Eindeutigkeit kann dabei z.B. durch atriale Extrasystolen oder einen atrialen Exitblock verursacht sein, der nur intermittierend auftritt.

Alternative Frequenzanalyse:

Um das zeitliche Fenster für die Wahrnehmung und Auswertung der atrialen Signale zu vergrößern, kann das beschriebene Verfahren alternativ zu dem in den fünf vorausgehenden Absätzen beschriebenen Verfahren auch im Rahmen einer Frequenzanalyse durchgeführt werden. Auch diese ist in der Lage, die durch Cross-Talk und FarField hervorgerufenen atrialen Senseereignisse herauszufiltern, so dass sich auch messbare Signale innerhalb der atrialen Blankingzeit nach einem ventrikulären Stimulationsimpuls Vp (PVARP: post ventricular atrial blanking periode) berücksichtigen lassen. Sollten keine vom Ventrikel auf das Atrium retrograd übergeleiteten Signale festgestellt werden, so wird diese Frequenzanalyse mit allen Zweier-Kombinationen der im untersuchten Zeitraum atrial wahrgenommenen Signalen durchgeführt. Dabei werden die Intervalle zwischen allen Zweier-Kombinationen von Signalen mit einem Bandpass gefiltert. Dieser lässt Frequenzen im Bereich Basic_rate +- Toleranz passieren. Jedes Signal, dass dieses Filter in mindestens einer Kombination passiert, wird als nicht intrinsisches Signal eingeordnet, da es mit großer Wahrscheinlichkeit auf die Stimulation in Atrium oder Ventrikel zurückzuführen ist. Umgekehrt wird jedes Signal als Eigenaktion eingeordnet, das dieses Filter nicht passiert.

Sofern der atriale Eigenrhythmus im vorangehenden Wahrnehmungstest als stabil festgestellt wurde, lassen sich mit einer identischen Frequenzanalyse die als Eigenaktion klassifizierten Signale auf Konsistenz mit diesem Eigenrhythmus prüfen. Hierbei muss allerdings berücksichtigt werden, dass einzelne Eigenaktionen durch analoges Blanking nicht aufgezeichnet werden. Daher sind bei der Auslegung des Bandpass-Filters neben dem Eigenrhythmus auch die entsprechenden niedergradigen subharmonischen Frequenzen zu berücksichtigen.

Die zweite Testphase zur Bestimmung der atrialen Reizschwelle:

Wenn in einer besonders bevorzugten Ausführungsvariante aller hier beschriebenen Elektrostimulatoren oder Verfahren die atriale Reizschwelle bestimmt werden soll, wird die beschriebene zweite Testphase (zur Wirksamkeitsprüfung der Vorhofstimulation) mit jeweils dekrementierter atrialer Impulsenergie so oft wiederholt, bis aus einer eindeutig wirksamen Impulsbeantwortung eine eindeutig unwirksame Impulsbeantwortung wird oder sich nach den obigen Kriterien eine eindeutige Feststellung nicht treffen lässt.

Um ein schnelles Erreichen der Reizschwelle zu gewährleisten, kann die Amplitude alternativ auch in nicht in konstanten 0.1 V Schritten dekrementiert werden, sondern mit folgender Formel bestimmt werden:

Wenn vorheriger Stimulus effektiv:

$$Amp(t+1)+Amp(t)+abs(Amp(t)-Amp(t-1))/2$$

Wenn vorheriger Stimulus ineffektiv:

$$Amp(t+1)=Amp(t)+abs(Amp(t)-Amp(t-1))/2$$

Durch diese Steuerung der Amplitude wird die Reizschwelle bei einer Startamplitude von z.B.3,6 V innerhalb von maximal 6 Messungen erreicht. Die niedrigste atriale Impulsenergie, bei der sich eine atriale Impulsbeantwortung feststellen lässt, gilt dann als die atriale Reizschwelle.

Die Erfindung soll nun anhand eines Ausführungsbeispiels mit Bezug auf die Figuren näher erläutert werden. Von den Figuren zeigt:

Fig.1: eine schematische Darstellung eines ZweikammerHerzschrittmachers mit Elektroden für das Atrium und den Ventrikel eines Herzens,

Fig. 2: eine schematische Blockdarstellung des Herzschrittmaches aus Figur 1 mit den im Zusammenhang mit der hier beschriebenen Funktion wesentlichen Bestandteilen,

Fig.3: ein Flussdiagramm, welches das Verhalten des Herzschrittmachers aus Figuren 1 und 2 im Falle der automatischen Bestimmung der atrialen Reizschwelle wiedergibt,

Fig.4: ein Flussdiagramm, welches die atriale Capture Detektion des Herzschrittmachers aus den Figuren und 2 wiedergibt, und

Fig. 5a und b: Zeit-Ereignisdiagramme, die das Verhalten des Herzschrittmachers aus Fig. 1 im Falle einer Fernfeldwahrnehmung ventrikulärer Ereignisse im Atrium widerspiegeln.

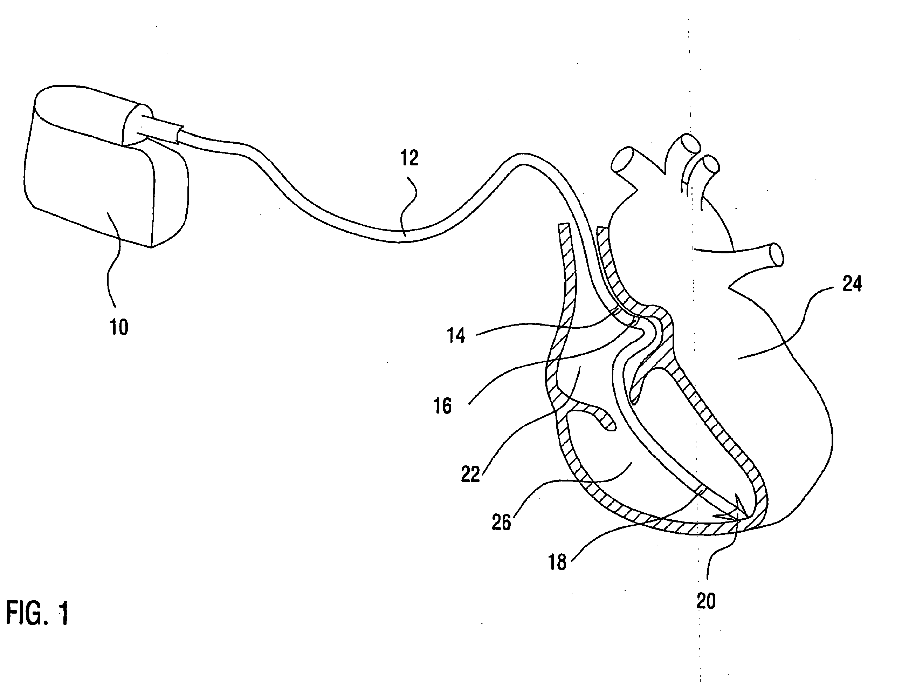

Figur 1 zeigt als implantierbares Stimulationsgerät einen Herzschrittmacher 10, an den eine Elektrodenleitung 12 angeschlossen ist, die zwei atriale Elektroden 14 und 16 und zwei ventrikuläre Elektroden 18 und 20 im Bereich ihres distalen Endes besitzt. In Figur 1 sind die atrialen Elektroden 14 und 16 im Atrium 22 eines Herzens 24 angeordnet, während die ventrikulären Elektroden 18 und 20 im Ventrikel 26 des Herzens 24 angeordnet sind. Anstelle einer gemeinsamen Elektrodenleitung 12 für Atrium und Ventrikel kann auch für das Atrium und den Ventrikel jeweils eine Elektrodenleitung mit beispielsweise zwei Elektroden vorgesehen sein. Im dargestellten Beispiel ist das implantierbare Stimulationsgerät ein Herzschrittmacher. Das Gerät könnte genauso gut ein Kardioverter oder Defibrillator sein. Genauso könnte der Herzschrittmacher 10 auch als biventrikulärer Herzschrittmacher mit einer Elektrodenleitung zur Stimulation des linken Ventrikels eines Herzens ausgebildet sein.

Wesentliche Bestandteile im Inneren des Herzschrittmachers 10 sind eine ventrikuläre Stimulationseinheit 30 und eine atriale Stimulationseinheit 32, die über jeweils zwei ventrikuläre Elektrodenleitungsanschlüsse 34 und 36 beziehungsweise zwei atriale Elektrodenleitungsanschlüsse 38 und 40 mit den entsprechenden Elektroden 14, 16, 18 und 20 der Elektrodenleitung 12 verbunden sind.

Mit den Elektrodenleitungsanschlüssen 34, 36, 38 und 40 sind außerdem ein atrialer Eingangsverstärker 42 beziehungsweise ein ventrikulärer Eingangsverstärker 44 verbunden. Die Stimulationseinheiten 30 und 32 sind so ausgebildet, dass sie Stimulationsimpulse mit einstellbarer Stimulationsimpulsstärke über die Elektrodenleitungsanschlüsse an die entsprechenden Elektroden der Elektrodenleitung 12 im Herzen 24 abgeben können.

Die Stimulationseinheiten 30 und 32 sowie die Eingangsverstärker 42 und 44 sind jeweils mit einer Stimulationssteuereinheit 50 verbunden, die beispielsweise eine Mikroprozessorsteuerung sein kann und Module für die Stimulations-Erfolgserfassung (Capture Detection) sowie für das Auslösen von Stimulationsimpulsen im Atrium und im Ventrikel und für das Einstellen der entsprechenden Stimulationsimpulsstärke aufweist. Dementsprechend weist die Stimulationssteuereinheit 50 einen Capture Detector 52 in Form eines Stimulationserfolgs-Erfassungsmoduls auf, sowie eine ventrikuläre Stimulationsimpulsauslösereinheit 54 sowie eine atriale Stimulationsimpulsauslösereinheit 56 und eine ventrikuläre Stimulationsstärkeneinheit 58 und eine atriale Stimulationsstärkeneinheit 60.

Der Capture Detector 52 ist derart mit den übrigen Modulen der Stimulationssteuereinheit 52 verbunden, dass diese auf die nachfolgend beschriebene Weise zusammenwirken

Im Rahmen der folgenden Beschreibung wird die folgende Terminologie verwendet:

LOC     bezeichnet ein Loss-of-Capture-Signal, welches bei unwirksamer (atrialer) Stimulation erzeugt wird

CAP     bezeichnet ein Capture-Signal, welches bei wirksamer (atrialer) Stimulation erzeugt wird

SR     bezeichnet den Wert der natürlichen Herzrate (Sinus Rhythmus)

PR     bezeichnet die jeweils aktuelle Stimulationsrate (Pacing Rate)

$R_{Test}$     bezeichnet die Stimulationsrate während eines (atrialen) Reizschwellentestes oder einer automatischen atrialen Stimulationserfolgskontrolle.

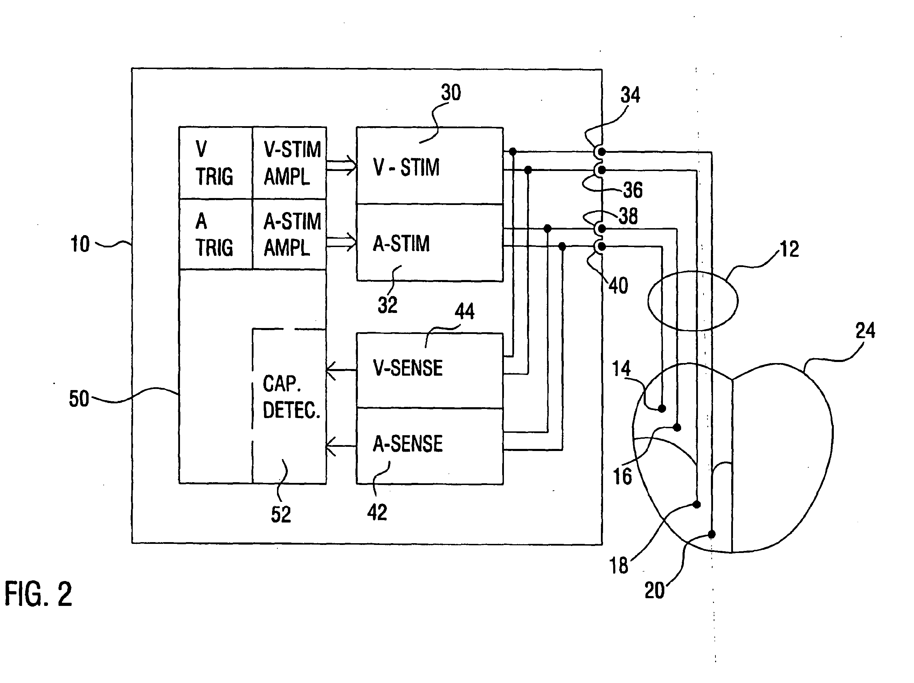

Nun soll das Verhalten des Schrittmachers aus Figur 1 während einer automatischen atrialen Stimulationserfolgskontrolle oder während eines atrialen Reizschwellentests beschrieben werden, siehe auch Figur 2:

Falls die anliegende Stimulationsrate (PR) vor dem vorgegebenen Zeitraum für die atriale Stimulationserfolgskontrolle niedriger ist, als die intrinsische Rate (SR), d.h. falls atriale Stimulationsimpulse regelmäßig inhibiert werden, ist die einzustellende Stimulationsrate ($R_{test}$) während des vorgegebenen Zeitraums für die atriale Stimulationserfolgskontrolle:

$$R_{Test} = SR + 10\ ppm$$

Falls die anliegende Stimulationsrate (PR) vor dem vorgegebenen Zeitraum für die atriale Stimulationserfolgskontrolle größer ist, als die intrinsische Rate (SR), d.h. falls atriale Stimulationsimpulse regelmäßig nicht inhibiert, sondern tatsächlich ausgelöst werden, ist die einzustellende Stimulationsrate ($R_{test}$) während des vorgegebenen Zeitraums für die atriale Stimulationserfolgskontrolle:

$$R_{Test} = PR + 10\ ppm$$

Während der automatischen atrialen Stimulationserfolgskontrolle werden folgen Betriebsparameter eingestellt:

Stimulationsmodus:                                       **DDD,**

Basisstimulationsrate =                                  **$R_{Test}$**

<div align="center">

**Atrio-ventrikuläre Verzögerungszeit (AVD) =          130 ms;**

</div>

Diese atrio-ventrikuläre Verzögerungszeit vermeidet ventrikuläre fusion-beats und retrograde ventriko-atriale Reizleitung nach wirksamen Ap-Vp)

<div align="center">

**Atriale Refraktärzeit (ARP) =                        $60000/R_{Test}$**

**[ms];**

</div>

Die atrio-ventrikuläre Verzögerungszeit wird nach intrinsischer Depolarisation nicht gestartet

| | |
|---|---|
| BiV | Off; |
| AT Prevention | Off, |
| ALC | Off, |
| MS | Off; |
| PMT-Detection | Off |

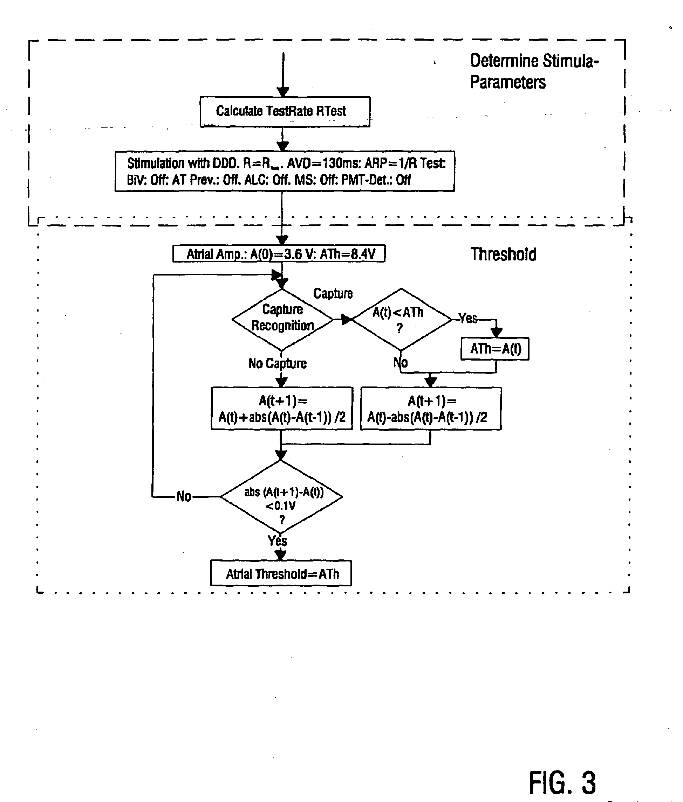

Nachdem die Stimulationsparameter auf diese Weise festegelegt sind, erfolgt das Einstellen der atrialen Stimulationsimpulsstärke (siehe Block "threshold" in Figur 3). Die die atriale Stimulationsimpulsstärke vorgebende atriale Amplitude wird auf einen Ausgangswert A (0) von 3,6 Volt festgelegt. Außerdem wird ein Grenzwert für die atriale Amplitude ATh von 8,4 Volt festgelegt.

Anschließend erfolgt die weiter hinten näher beschriebene Stimulationserfolgskontrolle (Capture Recognition). Ergibt diese, dass die atriale Stimulation erfolgreich war (Capture) wird geprüft, ob die jeweils aktuelle atriale Amplitude A(t) kleiner ist als die atriale Grenzamplitude ATh. Falls dies der Fall ist, wird die atriale Grenzamplitude ATh gleich der jeweils aktuellen atrialen Amplitude A(t) gesetzt. Ist hingegen die aktuelle atriale Amplitude A(t) kleiner als die atriale Grenzamplitude ATh wird der zuvor geltenden Wert für die atriale Grenzamplitude ATh (beispielsweise 8,4 Volt) beibehalten. In beiden Fällen erfolgt auf das Detektieren eines Stimulationserfolgs (Capture) hin eine Absenkung der atrialen Amplitude für die nächste Stimulation A(t+1) gemäß der folgenden Formel:

<div align="center">

**$A(t+1) = A(t) - abs (A(t)-A(t-1)) / 2$**

</div>

Ergibt die Stimulationserfolgskontrolle (Capture Recognition) jedoch nicht, dass ein Stimulationserfolg vorliegt (no Capture), erfolgt eine Erhöhung der atrialen Amplitude für die nächste Stimulation A(t+1) gemäß der folgenden Formel

<div align="center">

**$A(t+1) = A(t) + abs (A(t)-A(t-1)) / 2$**

</div>

Nach einem Erhöhen oder einem Absenken der atrialen Amplitude für die nächste Stimulation A(t+1) wird ein Test durchgeführt, ob der Absolutwert der Änderung der atrialen Amplitude kleiner ist al 0,1 Volt. Falls dies der Fall ist, gilt die jeweils aktuelle atriale Grenzamplitude ATh als atriale Reizschwelle, andernfalls wird ein neuer Reizschwellentest mit der durch das zuvor beschriebene Verfahren ermittelten jeweils aktuellen atrialen Amplitude A(t+1) durchgeführt, und zwar so lange, bis der Absolutwert der Amplitudenänderung kleiner ist als 0,1 Volt.

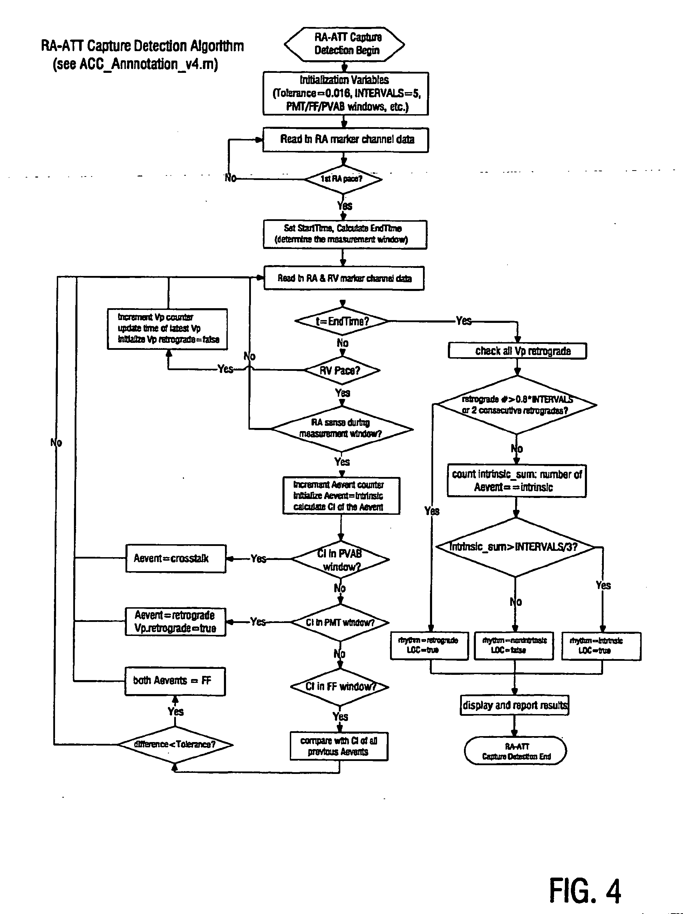

Die Details der Betriebsweise des atrialen Capture Detectors 52 können dem Flussdiagramm in Figur 4 entnommen werden.

Die im Flussdiagramm in Figur 4 dargestellte atriale Stimulationserfolgskontrolle (Atrial Capture Detection) nutzt eine Fähigkeit des hier beschriebenen Herzschrittmachers, nämlich atriale und ventrikuläre Ereignisse in Form von Stimulationsereignissen und Sense-Ereignissen (erfassten atrialen und ventrikulären Kontraktionen) und den jeweiligen Zeitpunkt des Auftretens eines Ereignisses zu erfassen und darzustellen. Jedem Ereignis wird somit ein Marker auf einem Zeitstrahl zugeordnet. Die zeitliche Abfolge der Ereignisse lässt sich anhand der jeweiligen Marker leicht erfassen.

Der Ablauf der atrialen Stimulationserfolgskontrolle gemäß dem Flussdiagramm in Figur 4 ist wie folgt:

Zunächst werden die folgenden Variablen initialisiert, nämlich ein Toleranzwert für ein Capture-Intervall, welches die Zeitdauer zwischen einem rechtsventrikulären Stimulationsimpuls und einem atrialen Ereignis widerspiegelt. Der Toleranzwert dient der später noch erläuterten Erkennung solcher atrialer Ereignisse, die auf Fernfeldwahrnehmung ventrikulärer Stimulationsimpulse beruhen. Außerdem wird eine Intervallzahl (intervals) vorgegeben, die beispielsweise zu 5 gesetzt wird. Außerdem sind die folgenden Zeitintervalle, nämlich PMT-Intervalle, Farfield-Intervall und postventrikuläre atriale Blankingzeit (PVAB) vorzugeben.

Nach dieser Initialisierung wird im rechtsatrialen Markerkanal nach einem ersten rechtsatrialen Stimulationsereignis (rechtsatrialer Stimulationsimpuls) gesucht. Mit diesem wird ein Messzeitfenster für die atriale Stimulationserfolgskontrolle gestartet.

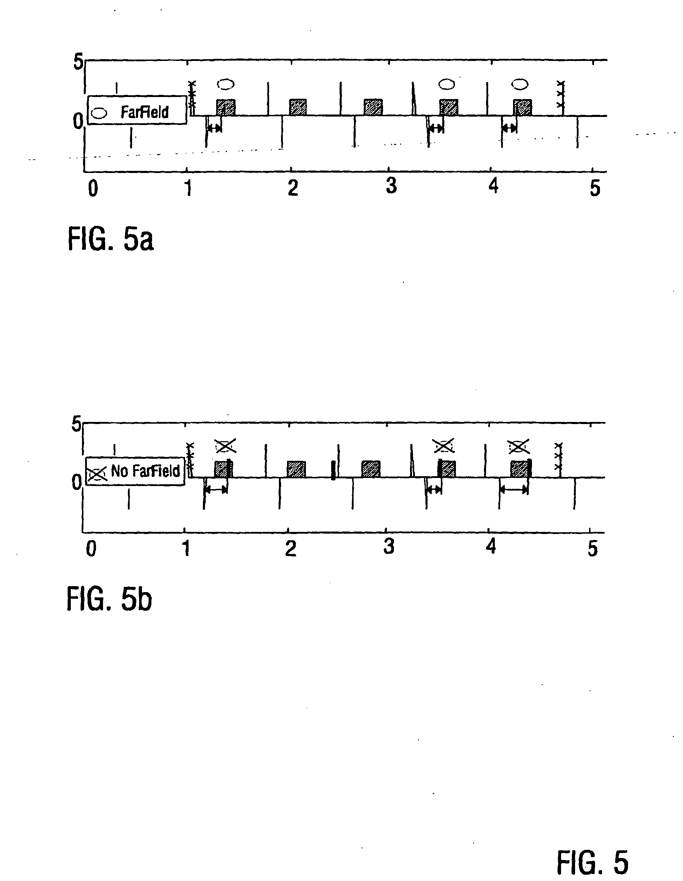

Innerhalb dieses Messzeitfensters wird zunächst geprüft, ob der Endzeitpunkt des Messzeitfensters erreicht ist. Falls ja, erfolgt die Endauswertung der Stimulationserfolgskontrolle, falls nein wird der Markerkanal daraufhin überprüft, ob ein rechtsventrikuläres Stimulationsereignis vorliegt (Die Begriffe Stimulationsereignis und Stimulationsimpuls werden im Zusammenhang mit der Beschreibung zu Figur 5 synonym verwendet):. Mit jedem vorliegenden rechtsventrikulären Stimulationsimpuls wird zunächst ein Zähler für rechtsventrikuläre Stimulationsimpulse (Vp) um 1 erhöht und geprüft, ob ein rechtsatriales Sense-Ereignis innerhalb des Messfensters vorliegt. Falls dies der Fall ist, wird ein atrialer Ereignis-Zähler um 1 erhöht und ein Capture Intervall für dieses atriale Ereignis berechnet. Das Capture-Intervall (CI) ist der zeitliche Abstand zwischen dem zuvor erfassten rechtsventrikulären Stimulationsereignis und dem daraufhin erfassten rechtsatrialen Sense-Ereignis. Anschließend wird geprüft, ob das Capture-Intervall innerhalb der postventrikulären atrialen Blankingzeit liegt, das heißt, ob das erfasste atriale Ereignis innerhalb der von dem rechtsventrikulären Stimulationsimpuls ausgehenden postventrikulären atrialen Blanking-Zeit liegt. Falls dies der Fall ist, wird das erfasste atriale Ereignis (Aevent) als Crosstalk-Ereignis kategorisiert, indem einer Variablen Aevent der wert "crosstalk" zugewiesen wird.

Falls das Capture-Intervall nicht in der postventrikulären atrialen Blankingzeit (PVAB) liegt, wird geprüft, ob das Capture-Intervall - das heißt das erfasste atriale Ereignis - in einem PMT-Intervall liegt. Falls dies der Fall ist, wird das atriale Ereignis als auf retrograder Reizleitung beruhendes Ereignis kategorisiert, indem der Variablen Aevent der Wert "retrograd" zugewiesen wird. Der zuvor erfasste ventrikuläre Stimulationsimpuls wird ebenfalls "retrograd" klassifiziert.

Falls das Capture-Intervall weder in der postventrikulären atrialen Blankingzeit noch im PMP-Intervall endet, wird geprüft, ob das Capture-Intervall im Farfield-Intervall (FF window) endet, das heißt ob das erfasste atriale Ereignis im Farfield-Intervall liegt. Falls dies der Fall ist, wird das Capture-Intervall mit allen vorangegangenen Capture-Intervallen der zuvor erfassten atrialen Ereignisse verglichen. Wenn sich wenigstens zwei dieser Capture-Intervalle um eine Zeitdauer unterscheiden, die kleiner ist als der zuvor festgelegte Toleranzwert, werden die beiden zu den entsprechenden Capture-Intervallen gehörenden atrialen Ereignisse als auf Fernfeldwahrnehmung (FF) beruhend kategorisiert, indem der Variablen Aevent der Wert "farfield" oder "FF" zgewiesen wird.

Wenn das Messzeitfenster beendet ist, das heißt wenn die anfängliche Prüfung ergeben hat, dass der Endzeitpunkt des Messzeitfensters erreicht ist, erfolgt die Auswertung der Stimulationserfolgskontrolle. Dazu wird zunächst die Zahl der erfassten mit "retrograd" klassifizierten ventrikulären Stimulationsereignisse ermittelt und geprüft, ob diese größer ist, als 0,8 mal die zuvor festgelegte Intervallzahl (intervals) oder ob zwei unmittelbar aufeinander folgende, mit "retrograd" klassifizierte ventrikuläre Stimulationsimpulse vorliegen. Falls eins von beidem oder beides der Fall ist, wird eine Variable Rhythmus (Rhythm) auf "retrograd" gesetzt. Außerdem wird ein Loss-of-Capture (LOC)-Signal erzeugt, indem eine Variable LOC auf "wahr" gesetzt wird.

Falls keine der beiden zuvor genannten Bedingungen bezüglich der erfassten ventrikulären Stimulationsereignisse erfüllt ist, wird die Zahl der erfassten intrinsischen atrialen Ereignisse bestimmt; dies sind all diejenigen in dem Messzeitfenster erfassten atrialen Ereignisse, die nicht mit "Crosstalk", "retrograd" oder "Ferfeld" kategorisiert sind. Ist die Zahl der intrinsischen atrialen Ereignisse größer als der Intervallwert (Intervals), geteilt durch 3, wird der Variablen Rhythmus (Rhythm) der Wert "intrinsisch" zugeordnet. Gleichzeitig wird die Variable Loss-of Capture-(LOC) auf "wahr" gesetzt, das heißt, es wird ein Loss-of-Capture-Signal erzeugt.

Ist die Summe der intrinsischen atrialen Ereignisse kleiner oder gleich der Intervallzahl geteilt durch 3, wird die Variable "Rhythmus" auf "nichtintrinsisch" gesetzt und die Variable LOC auf "nicht wahr". Dieser Fall kennzeichnet die erfolgreiche atriale Stimulation, bei der ein intrinsischer atrialer Rhythmus unterdrückt wird.

Nach diesen Überprüfungen im Rahmen der Auswertung der atrialen Stimulationserfolgs-Kontrolle wird das Ergebnis, das heißt der Wert der jeweiligen Variable "Rhythmus" und der Variablen LOC ausgegeben.

In den Figuren 5a und b ist anhand der dort dargestellten Zeit-EreignisDiagramme das Erkennen solcher atrialer Ereignisse dargestellt, die auf Fernfeldwahrnehmung beruhen. Oberhalb der horizontalen Mittellinie des jeweiligen Diagramms ist jeweils in Hellgrau das jeweilige Fernfeld-Intervall dargestellt, in dem ein atriales Ereignis liegen muss, um überhaupt als auf Fernfeldwahrnehmung beruhend in Frage zu kommen.

Die atrialen Ereignisse sind oberhalb der horizontalen Mittellinie in den Diagrammen dargestellt. Unterhalb der horizontalen Mittellinie sind die ventrikulären Stimulationsereignisse dargestellt.

Zweite Bedingung dafür, dass ein atriales Ereignis als auf Fernfeldwahrnehmung beruhend kategorisiert wird (neben der Bedingung dass das atriale Ereignis in dem Fernfeld-Intervall auftritt) ist, dass der zeitliche Abstand des atrialen Ereignisses zum vorangegangenen ventrikulären Ereignis innerhalb enger Toleranzgrenzen dem zeitlichen Abstand eines anderen atrialen Ereignisses zum jeweils vorangegangenen ventrikulären Stimulationsimpuls entspricht. Der zeitliche Abstand zwischen ventrikulären Stimulationsimpulsen und dem erfassten atrialen Ereignis - zuvor auch als Capture-Intervall (CI) bezeichnet - ist in den Figuren 5a und b jeweils durch einen Doppelpfeil dargestellt.

Da in dem in Figur 5a dargestellten Beispiel drei atriale Ereignisse erfasst wurden, die sowohl im Fernfeld-Intervall (grau dargestellt) liegen, als auch jeweils praktisch den gleichen Abstand zum vorangegangenen ventrikulären Stimulationsimpuls haben, werden die drei in Figur 5a entsprechend markierten atrialen Ereignisse als auf Fernfeldwahrnehmung beruhend kategorisiert.

Bei dem Beispiel in Figur 5b ist zwar ebenfalls die Bedingung erfüllt, dass die entsprechenden atrialen Ereignisse innerhalb des Fernfeld-Intervalls liegen, jedoch unterscheiden sich deren Abstände zu jeweils vorangegangenen ventrikulären Stimulationsimpuls derart voneinander, dass die atrialen Ereignisse nicht als auf Fernfeldwahrnehmung beruhend kategorisiert werden.

Diese Form der Erkennung solcher atrialen Ereignisse, die auf Fernfeldwahrnehmung beruhen, macht es möglich, die postventrikuläre atriale Blankingzeit (PVAB) besonders kurz zu halten, so dass auch eine besonders große Zahl von intrinsischen atrialen Ereignissen zu erfassen ist, falls die atriale Stimulation wirkungslos ist, weil beispielsweise die Stimulationsimpulsstärke des atrialen Stimulationsimpulses (atriale Amplitude) geringer ist, als die atriale Reizschwelle. In diesem Fall wirkungsloser atrialer Stimulation ergibt sich ein regelmäßiger atrialer Rhythmus unabhängig vom atrialen Stimulationsrhythmus. Auch dies ist der Figur 5b zu entnehmen. Dort sind oberhalb der horizontalen Mittellinie sowohl atriale Stimulationsereignisse aufgetragen, und zwar in Form langer vertikaler Marker, während atriale Sense-Ereignisse in Form kurzer, breiter atrialer Marker, jeweils oberhalb der horizontalen Mittellinie des Diagramms dargestellt sind.

Die Vorteile die mit der Erfindung zu erzielen sind, ergeben sich somit aus den Zeitereignis-Diagrammen in den Figuren 5a und b in anschaulicher Art und Weise.

**Patentansprüche**

1. Implantierbarer Elektrostimulator für die Stimulation eines Herzens, insbesondere Herzschrittmacher für wenigstens zwei Herzkammern, mit wenigstens einem atrialen und einem ventrikulären Kanal,
mit Elektrodenanschlüssen, die mit einer Stimulationseinheit zum Abgeben atrialer und ventrikulärer Stimulationsimpulse derart verbunden sind, dass über eine oder mehrere an die Elektrodenanschlüsse angeschlossene Elektrodenleitungen atriale oder ventrikuläre Stimulationsimpulse abzugeben sind, sowie
mit einer Stimulationssteuereinheit, die mit der Stimulationseinheit verbunden und ausgebildet ist, die Abgabe von Stimulationsimpulsen auszulösen und die Stärke der Stimulationsimpülse zu bestimmen und hierzu eine Stimulationssteuersignal an die Stimulationseinheit abzugeben und
mit einer Detektionseinheit, die mit der Stimulationssteuereinheit und wenigstens indirekt mit den Elektrodenanschlüssen verbunden und ausgebildet ist, an den Elektrodenanschlüssen anliegende, insbesondere intracardial aufzunehmende elektrische Potentiale auszuwerten, um für natürliche Kontraktionen eines Atriums charakteristische intrinsische atriale Ereignisse zu detektieren,
wobei die Stimulationssteuereinheit ausgebildet ist,
zum Zwecke der Stimulationserfolgskontrolle (Capture-Detektion) im Atrium wenigstens einen atrialen Stimulationsimpuls auszulösen, und einen Stimulationserfolg (Capture) im Atrium oder eine unwirksame atriale Stimulation (Non-capture) anhand des Nicht-Detektierens beziehungsweise des Detektierens wenigstens eines gegebenenfalls auftretenden intrinsischen (natürlichen, spontanen) atrialen Ereignisses innerhalb eines vorgegebenen Zeitraums zu erfassen,
**dadurch gekennzeichnet, dass** Stimulationssteuereinheit weiter dazu ausgebildet ist,
wenigstens für einen begrenzten, insbesondere der Stimulationserfolgskontrolle (Capture-Detektion) oder einem Reizschwellentest dienenden, vorgegebenen, mehrere Herzzyklen einschließenden Zeitraum ventrikuläre Stimulationsimpulse mit Ablauf eines mit einem intrinsischen oder stimulierten atrialen Ereignis beginnenden AV-Intervalls auszulösen, welches derart kurz ist, dass Fusionsereignisse, d.h. Ereignisse bei denen ein ventrikulärer Stimulationsimpuls im wesentlichen zeitgleich mit einer natürlichen ventrikulären Kontraktion erfolgt, vermieden werden, und
über den vorgegebenen Zeitraum Stimulationsimpulse mit einer eingestellten Stimulationsrate und/oder in einem getriggerten Modus derart auszulösen, dass Stimulationsimpulse nicht inhibiert werden, und
atriale Ereignisse, die auf Fernfeldwahrnehmung ventrikulärer Ereignisse im Atrium beruhen von intrinsischen atrialen Ereignissen zu unterscheiden, indem solche atrialen Ereignisse, die einen Zeitabstand zum jeweils vorangehenden ventrikulären Stimulationsimpuls haben, der innerhalb enger Toleranzgrenzen dem zeitlichen Abstand eines anderen atrialen Ereignisses zum jeweils vorangegangenen ventrikulären Stimulationsimpuls entspricht, als auf Fernfeldwahrnehmung basierend gewertet und nicht als intrinsische Ereignisse gezählt werden, und

für den vorgegebenen Zeitraum gegebenenfalls auftretende intrinsische (natürliche, spontane) atriale Ereignisse zu detektieren und

für den vorgegebenen Zeitraum auf Basis der detektierten oder nichtdetektierten intrinsischen (natürliche, spontane) atriale Ereignisse das Vorliegen oder Nichtvorliegen eines natürlichen Herzrhythmus zu ermitteln.

2. Elektrostimulator nach Anspruch 1, **dadurch gekennzeichnet, dass** die Stimulationssteuereinheit ausgebildet ist, ein ein auf Fernfeldwahrnehmung beruhendes atriales Ereignis kennzeichnendes Farfield-Signal zu erzeugen, wenn das von der Detektionseinheit erfasste atriale Ereignis in ein vorgegebenes Farfeld-Koppelintervall fällt, welches ausgehend von einem jeweiligen ventrikulären Stimulationsimpuls gestartet wird und welches spätestens 150 ms nach dem Auslösen eines ventrikulären Stimulationsimpulses endet.

3. Elektrostimulator nach Anspruch 2, **dadurch gekennzeichnet, dass** das Farfield-Koppelintervall 50 bis 100 ms nach Auslösen des ventrikulären Stimulationsimpulses gestartet wird und 150 bis 200 ms nach dem Auslösen desselben ventrikulären Stimulationsimpulses endet.

4. Elektrostimulator nach einem der Ansprüche 1 bis 3, **dadurch gekennzeichnet, dass** die Stimulationssteuereinheit ausgebildet ist, die AV-Zeit während des vorgegebenen Zeitraums derart einzustellen, dass die AV-zeit so kurz ist, dass keine Fusionsschläge im Ventrikel auftreten und keine retrograden Leitungen vom Ventrikel zum Atrium bei effektiver atrialer und ventrikulärer Stimulation auftreten.

5. Elektrostimulator nach Anspruch 4, **dadurch gekennzeichnet, dass** das AV-Intervall kürzer ist als 200ms und vorzugsweise eine Dauer zwischen 100 und 130 ms hat.

6. Elektrostimulator nach einem der Ansprüche 1 bis 5, **dadurch gekennzeichnet, dass** die Stimulationssteuereinheit ausgebildet ist, auch Ereignisse innerhalb einer Blankingzeit nach ventrikulärer Stimulation derart auszuwerten, dass all diejenigen Ereignisse innerhalb der Blankingzeit dann als intrinsisches Ereignis kategorisiert werden, zu denen kein anderes atriales Ereignis innerhalb des vorgegebenen Zeitraums existiert, welches mit einem gleichen oder ähnlichen Koppelintervall auf einen jeweils vorangehenden ventrikulären Stimulationsimpuls folgt oder folgte.

7. Elektrostimulator nach einem der Ansprüche 1 bis 6, **dadurch gekennzeichnet, dass** Stimulationssteuereinheit dazu ausgebildet ist, ein Retrograde-Reizleitungs-Signal zu erzeugen, falls die Detektionseinheit ein intrinsisches atriales Ereignis innerhalb eines vorgegebenen zeitlichen Abstandes nach Auslösen eines ventrikulären Stimulationsimpulses detektiert.

8. Elektrostimulator nach Anspruch 7, **dadurch gekennzeichnet, dass** der vorgegebene zeitliche Abstand ein Koppelintervall von 180 ms bis 350 ms zwischen Auslösen des ventrikulären Stimulationsimpulses und Detektieren des intrinsischen atrialen Ereignisses ist.

9. Elektrostimulator nach Anspruch 7 oder 8, **dadurch gekennzeichnet, dass** Stimulationssteuereinheit dazu ausgebildet ist, ein Loss-of-Capture-Signal (LOC) zu erzeugen, falls das Verhältnis der Anzahl von Retrograden-Reizleitungs-Signalen zur Anzahl ausgelöster ventrikulärer Stimulationsimpulse innerhalb eines vorgegebenen Zeitraums eine vorgegeben Schwellenwert zwischen 0,5 und 1 überschreitet.

10. Elektrostimulator nach Anspruch 9, **dadurch gekennzeichnet, dass** der vorgegebene Schwellenwert 0,8 beträgt.

11. Elektrostimulator nach Anspruch 1, **dadurch gekennzeichnet, dass** Stimulationssteuereinheit dazu ausgebildet ist, ein Crosstalk-Signal zu erzeugen, falls die Detektionseinheit ein intrinsisches atriales Ereignis innerhalb eines vorgebenen Zeitfensters detektiert, das mit Auslösen eines jeweiligen ventrikulären Stimulationsimpulses beginnt.

12. Elektrostimulator nach Anspruch 11, **dadurch gekennzeichnet, dass** das vorgegebene Zeitfenster eine Dauer von 30 ms bis 70 ms, vorzugsweise 50 ms hat.

13. Elektrostimulator nach Anspruch 11 oder 12, **dadurch gekennzeichnet, dass** die Detektionseinheit ausgebildet ist, nur solche atrialen Ereignisse als intrinsische Ereignisse zu detektieren, denen kein Crosstalk-Signal oder kein FarFeld-Signal zuzuordnen ist.

14. Elektrostimulator nach einem der Ansprüche 1 bis 13, **dadurch gekennzeichnet, dass** die Stimulationssteuereinheit ausgebildet ist, das Auslösen eines ventrikulären Stimulationsimpulses zu inhibieren, falls innerhalb des AV-Intervalls ein intrinsisches ventrikuläres Ereignis detektiert wird.

15. Elektrostimulator nach Anspruch 1, **dadurch gekennzeichnet, dass** die Stimulationssteuereinheit ausgebildet ist, aus einer vor dem vorgegebenen, mehrere Herzzyklen einschließenden Zeitraum anliegenden Rate die eingestellte Stimulationsrate so abzuleiten, dass die eingestellte Stimulationsrate größer ist, als die zuvor anliegende Rate.

16. Elektrostimulator nach Anspruch 1 oder 15, **dadurch gekennzeichnet, dass** die Stimulationssteuereinheit ausgebildet ist, die Stimulationsrate, bei der die Abgabe von Stimulationsimpulsen nicht inhibiert wird in einem inhibierten Modus durch wiederholtes Erhöhen der Stimulationsrate um einen vorbestimmten Wert, bis keine Inhibierung mehr auftritt, einzustellen.

17. Elektrostimulator nach Anspruch 1 oder 5, **dadurch gekennzeichnet, dass** die Stimulationssteuereinheit ausgebildet ist, die Stimulationsrate, bei der die Abgabe von Stimulationsimpulsen nicht inhibiert wird in einem getriggerten Modus durch einmaliges Erhöhen der Stimulationsrate, um einen vorbestimmten Wert zu bestimmen.

18. Elektrostimulator nach einem der Ansprüche 1 oder 17, **dadurch gekennzeichnet, dass** der Elektrostimulator als atrialer Herzschrittmacher, die Stimulationseinheit zum Erzeugen und Abgeben atrialer Stimulationsimpulse, die Detektionseinheit zum Erfassen intrinsischer atrialer Ereignisse und Stimulationssteuereinheit zum Ermitteln eines natürlichen atrialen Herzrhythmus auf Basis von der Detektionseinheit erfasster intrinsischer Ereignisse im Atrium ausgebildet sind.

19. Elektrostimulator nach Anspruch 18, **dadurch gekennzeichnet, dass** die Stimulationssteuereinheit ausgebildet ist, in dem vorgegebenen, mehrere Herzzyklen einschließenden Zeitraum atriale Stimulationsimpulse mit einer eingestellten Stimulationsrate in einem getriggerten Modus oder mit einer derart eingestellten atrialen Stimulationsrate auszulösen, dass atriale Stimulationsimpulse nicht inhibiert werden, und ventrikuläre Stimulationsimpulse mit einer eingestellten Stimuiätiönsräte in einem Demand-Modus auszulösen, bei dem ventrikuläre Stimulationsimpulse inhibiert werden, falls innerhalb eines jeweiligen Ventrikulären Escape-Intervalls intrinsische ventrikuläre Ereignisse erfasst werden.

20. Elektrostimulator nach einem der Ansprüche 1 bis 19, **dadurch gekennzeichnet, dass** die Stimulationssteuereinheit ausgebildet ist, das Vorliegen oder Nichtvorliegen eines natürlichen Herzrhythmus in Abhängigkeit davon zu ermitteln, ob und/oder wie viele intrinsische Ereignisse von der Detektionseinheit innerhalb des vorgegebenen Zeitraums detektiert werden.

21. Elektrostimulator nach einem der Ansprüche 1 bis 20, **dadurch gekennzeichnet, dass** die Stimulationssteuereinheit ausgebildet ist, für die Ermittlung des Vorliegens oder Nicht-Vorliegens eines natürlichen Herzrhythmus Ausblendzeiten (Blankingzeiten), in denen eventuell auftretende natürliche Ereignisse nicht erfasst werden können, zu berücksichtigen.

22. Elektrostimulator nach Anspruch 21, **dadurch gekennzeichnet, dass** die Stimulationssteuereinheit ausgebildet ist, anhand erfasster natürlicher Ereignisse unter Berücksichtigung potentiell in den Ausblendzeiten aufgetretener, nicht erfasster natürlicher Ereignisse einen natürlichen Herzrhythmus zu rekonstruieren und ein einen natürlichen Herzrhythmus kennzeichnendes Signal zu erzeugen, falls die erfassten natürlichen Herzereignisse einem regelmäßigen, natürlichen Herzrhythmus zuzuordnen sind.

23. Elektrostimulator nach einem der Ansprüche 1 bis 22, **dadurch gekennzeichnet, dass** die Detektionseinheit ausgebildet ist, für jedes detektierte intrinsischer Ereignis ein Sense-Markersignal in relativer zeitlicher Zuordnung mit Bezug auf den Zeitpunkt des Auftretens des Ereignisses zu erzeugen.

24. Elektrostimulator nach Anspruch 23, **dadurch gekennzeichnet, dass** die Stimulationssteuereinheit dazu ausgebildet ist, für jeden ausgelösten Stimulationsimpuls jeweils ein Pace-Markersignal zu erzeugen und das Vorliegen eines natürlichen Herzrhythmus auf Basis wenigstens der Sense-Markersignale oder zusätzlich der Pace-Markersignale zu ermitteln.

25. Elektrostimulator nach einem der Ansprüche 1 bis 24 **dadurch gekennzeichnet, dass** die Stimulationssteuereinheit ausgebildet ist, ein Stimulationssteuersignal für die Stimulationsstärke abhängig davon zu bestimmen, ob ein Vorliegen oder ein Nichtvorliegen eines natürlichen Herzrhythmus innerhalb des vorgegebenen Zeitraums ermittelt ist.

26. Elektrostimulator nach Anspruch 25 **dadurch gekennzeichnet, dass** die Stimulationssteuereinheit ausgebildet ist, ein Stimulationsteuersignal, welches eine Erhöhung der Impulsstärke eines Stimulationsimpulses bewirkt, dann zu erzeugen, wenn ein Vorliegen eines natürlichen Herzrhythmus innerhalb des vorgegebenen Zeitraums ermittelt ist.

27. Elektrostimulator nach einem der Ansprüche 1 bis 26 **dadurch gekennzeichnet, dass** die Stimulationssteuereinheit ausgebildet ist, ein Capture-Control-Signal in Abhängigkeit davon zu bestimmen, ob ein Vorliegen oder ein Nichtvorliegen eines natürlichen Herzrhythmus innerhalb des vorgegebenen Zeitraums ermittelt ist und zusätzlich oder alternativ im Falle des Vorliegens eines natürlichen Herzrhythmus innerhalb des vorgegebenen Zeitraums ein Loss-of-Capture-Signal (LOC) zu erzeugen.

28. Elektrostimulator nach Anspruch 1, **dadurch gekennzeichnet, dass** die Stimulationssteuereinheit dazu ausgebildet ist, mehrere Stimulationsimpulse mit der abgeleiteten, erhöhten Stimulationsrate abzugeben und ein Stimulationsteuersignal, welches eine Erhöhung der Impulsstärke eines Stimulationsimpulses bewirkt, dann zu erzeugen, wenn innerhalb des vorgegebenen Zeitraums das Verhältnis der Anzahl der mit der abgeleiteten, erhöhten Stimulationsrate abgegebenen Impulse zu einer Anzahl innerhalb des vorgegebenen Zeitraums detektierten intrinsischen Ereignisse einen vorgegebenen Wert überschreitet.

**Claims**

1. An implantable electrostimulator for stimulation of the heart, in particular a cardiac pacemaker for at least two chambers of the heart, having at least one atrial channel and one ventricular channel,
   having electrode terminals, which are connected to a stimulation unit for delivering atrial and ventricular stimulation pulses, such that atrial or ventricular stimulation pulses are to be delivered via one or more electrode lines connected to the electrode terminals, and
   having a stimulation control unit, which is connected to the stimulation unit and is designed to trigger the delivery of stimulation pulses and to determine the intensity of the stimulation pulses and to deliver a stimulation control signal to the stimulation unit in response thereto, and
   having a detection unit, which is connected to the stimulation control unit and at least indirectly to the electrode terminals and is designed to evaluate electric potentials, which are applied to the electrode terminals and are to be picked up intracardially in particular, to detect intrinsic atrial events that are characteristic of natural atrial contractions, the stimulation control unit being designed
   to trigger at least one atrial stimulation pulse for the purpose stimulation success monitoring (capture detection) in the atrium and to detect a stimulation success (capture) in the atrium or an ineffective atrial stimulation (non-capture) on the basis of the capture and/or non-capture of at least one intrinsic (natural, spontaneous) atrial event possibly occurring within a predetermined period of time,
   **characterized in that** the stimulation control unit is further designed
   to trigger ventricular stimulation pulses at the end of an AV interval beginning with an intrinsic or stimulated atrial event at least for a limited, prespecified period of time including several cardiac cycles serving in particular for stimulation success monitoring (capture detection) or a stimulus threshold test, said AV interval being short enough to prevent fusion events, i.e., events in which a ventricular stimulation pulse occurs essentially at the same time as a natural ventricular contraction, and
   to trigger stimulation pulses at a set stimulation rate over the prespecified period of time and/or in a triggered mode, such that stimulation pulses are not inhibited, and
   to differentiate atrial events based on far-field detection of ventricular events in the atrium from intrinsic atrial events by the fact that such atrial events which have an interval of time from the respective preceding ventricular stimulation pulse corresponding, within narrow tolerance limits, to the interval of time of another atrial event from the respective preceding ventricular stimulation pulse, is evaluated as based on far-field detection and not to be counted as intrinsic events, and
   to detect intrinsic (natural, spontaneous) atrial events possibly occurring for the prespecified period of time, and
   to ascertain the presence or absence of a natural cardiac rhythm for the prespecified period of time on the basis of the detected or non-detected intrinsic (natural, spontaneous) atrial events.

2. The electrostimulator according to claim 1,
   **characterized in that**
   the stimulation control unit is designed to generate a far-field signal characterizing an atrial event and based on a far-field detection when the atrial event detected by the detection unit falls in a prespecified far-field coupling interval, which is initiated starting from the respective ventricular stimulation pulse and which ends at the latest 150 ms after the triggering of a ventricular stimulation pulse.

3. The electrostimulator according to claim 2,
**characterized in that**
the far-field coupling interval starts 50 to 100 ms after triggering of the ventricular stimulation pulse and ends 150 to 200 ms after the triggering of the same ventricular stimulation pulse.

4. The electrostimulator according to any one of claims 1 to 3,
**characterized in that**
the stimulation control unit is designed to adjust the AV time during the prespecified period of time, such that the AV time is short enough that no fusion beats occur in the ventricle and no retrograde conduction from the ventricle to the atrium occurs with effective atrial and ventricular stimulation.

5. The electrostimulator according to claim 4,
**characterized in that**
the AV interval is shorter than 200 ms and preferably has a duration between 100 and 130 ms.

6. The electrostimulator according to any one of claims 1 to 5,
**characterized in that**
the stimulation control unit is designed to also evaluate events within a blanking time after ventricular stimulation, such that all the events within the blanking time are categorized as intrinsic events for which no other atrial event, which follows or followed a respective preceding ventricular stimulation pulse with the same or similar coupling interval, exists within the prespecified period of time.

7. The electrostimulator according to any one of claims 1 to 6,
**characterized in that**
the stimulation unit is designed to generate a retrograde stimulus conduction signal if the detection unit detects an intrinsic atrial event within a prespecified interval of time after triggering of a ventricular stimulation pulse.

8. The electrostimulator according to claim 7,
**characterized in that**
the prespecified interval of time is a coupling interval of 180 ms to 350 ms between triggering of the ventricular stimulation pulse and detection of the intrinsic atrial event.

9. The electrostimulator according to claim 7 or 8,
**characterized in that**
the stimulation control unit is designed to generate a loss-of-capture (LOC) signal if the ratio of the number of retrograde stimulus conduction signals to the number of triggered ventricular stimulation pulses within a prespecified period of time exceeds a prespecified threshold value between 0.5 and 1.

10. The electrostimulator according to claim 9,
**characterized in that**
the prespecified threshold value is 0.8.

11. The electrostimulator according to claim 1,
**characterized in that**
the stimulation control unit is designed to generate a crosstalk signal if the detection unit detects an intrinsic atrial event within a prespecified time window, beginning with triggering of a respective ventricular stimulation pulse.

12. The electrostimulator according to claim 11,
**characterized in that**
the prespecified time window has a duration of 30 ms to 70 ms, preferably 50 ms.

13. The electrostimulation according to claim 11 or 12,
**characterized in that**
the detection unit is designed to detect as intrinsic events only such atrial events to which no crosstalk signal or far-field signal is to be assigned.

**14.** The electrostimulator according to any one of claims 1 to 13,
**characterized in that**
the stimulation control unit is designed to inhibit the triggering of a ventricular stimulation pulse if an intrinsic ventricular event is detected within the AV interval.

**15.** The electrostimulator according to claim 1,
**characterized in that**
the stimulation control unit is designed to derive the set stimulation rate from a rate applied before the prespecified period of time including multiple cardiac cycles, such that the set stimulation rate is greater than the rate applied previously.

**16.** The electrostimulator according to claim 1 or 15,
**characterized in that**
the stimulation control unit is designed to adjust the stimulation rate at which the delivery of stimulation pulses is not inhibited in an inhibited mode by repeatedly increasing the stimulation rate by a predetermined value until inhibition no longer occurs.

**17.** The electrostimulator according to claim 1 or 5,
**characterized in that**
the stimulation control unit is designed to determine the stimulation rate at which the delivery of stimulation pulses is not inhibited in a triggered mode by increasing the stimulation rate once by a predetermined value.

**18.** The electrostimulator according to any one of claims 1 or 17,
**characterized in that**
the electrostimulator is designed as an atrial cardiac pacemaker, the stimulation unit is designed for generating and delivering atrial stimulation pulses, the detection unit is designed for detecting intrinsic atrial events, and the stimulation unit is designed for ascertaining a natural atrial cardiac rhythm on the basis of intrinsic events in the atrium detected by the detection unit.

**19.** The electrostimulator according to claim 18,
**characterized in that**
the stimulation unit is designed to trigger atrial stimulation pulses at a set stimulation rate in a triggered mode within the prespecified period of time including multiple cardiac cycles,
or at an atrial stimulation rate that is set such that atrial stimulation pulses are not inhibited, and
to trigger ventricular stimulation pulses at a set stimulation rate in a demand mode, in which ventricular stimulation pulses are inhibited if intrinsic ventricular events are detected within a respective ventricular escape interval.

**20.** The electrostimulator according to any one of claims 1 to 19,
**characterized in that**
the stimulation control unit is designed to ascertain the presence or absence of a natural cardiac rhythm as a function of whether and/or how many intrinsic events are detected by the detection unit within the prespecified period of time.

**21.** The electrostimulator according to any one of claims 1 to 20,
**characterized in that** the stimulation control unit is designed to take into account, in the determination of the presence or absence of a natural cardiac rhythm, blanking times during which any natural events possibly occurring cannot be detected.

**22.** The electrostimulator according to claim 21,
**characterized in that**
the stimulation control unit is designed to reconstruct a natural cardiac rhythm on the basis of detected natural events, taking into account natural events potentially occurring in the blanking intervals but not detected and to generate a signal characterizing a natural cardiac rhythm if the detected natural cardiac events are to be assigned to a regular natural cardiac rhythm.

**23.** The electrostimulator according to any one of claims 1 to 22,
**characterized in that**

the detection unit is designed to generate a sense marker signal in relative chronological correlation with respect to the point in time of occurrence of the event for each detected intrinsic event.

24. The electrostimulator according to claim 23,
    **characterized in that**
    the stimulation control unit is designed to generate a pace marker signal for each triggered stimulation pulse and to ascertain the presence of a natural cardiac rhythm on the basis of at least the sense marker signals or in addition to the pace marker signals.

25. The electrostimulator according to any one of claims 1 to 24,
    **characterized in that**
    the stimulation control unit is designed to determine a stimulation control signal for the stimulation intensity as a function of whether or not the presence of a natural cardiac rhythm is detected within the prespecified period of time.

26. The electrostimulator according to claim 25,
    **characterized in that**
    the stimulation control unit is designed to induce an increase in the pulse intensity of a stimulation pulse when the presence of a natural cardiac rhythm within the prespecified period of time is detected.

27. The electrostimulator according to any one of claims 1 to 26,
    **characterized in that**
    the stimulation control unit is designed to determine a capture control signal as a function of whether or not the presence of a natural cardiac rhythm is detected within the prespecified period of time and additionally or alternatively to generate a loss-of-capture (LOC) signal in the event of occurrence of a natural cardiac rhythm within the prespecified period of time.

28. The electrostimulator according to claim 1,
    **characterized in that**
    a stimulation control unit is designed to deliver multiple stimulation pulses at with the derived increased stimulation rate and to generate a stimulation control signal, which causes an increase in the pulse intensity of a stimulation pulse when the ratio of the number of pulses delivered at the derived increased stimulation rate to the number of intrinsic events detected within the prespecified period of time exceeds a prespecified value.

**Revendications**

1. Stimulateur électrique implantable pour la stimulation d'un coeur, notamment stimulateur cardiaque pour au moins deux chambres du coeur, comprenant au moins un canal atrial et un canal ventriculaire,
   doté de raccordements électriques qui sont reliés à une unité de stimulation destinée à émettre des impulsions de stimulation atriales et ventriculaires de manière à ce que des impulsions de stimulation atriales ou ventriculaires soient émises via une ou plusieurs conducteurs à électrode raccordés aux raccordements électriques, ainsi que d'une unité de commande de stimulation qui est reliée à l'unité de stimulation et conçue pour déclencher l'émission d'impulsions de stimulation et définir l'intensité des impulsions de stimulation et émettre pour ce faire un signal de commande de stimulation vers l'unité de stimulation et
   d'une unité de détection qui est reliée à l'unité de commande de stimulation et au moins indirectement aux raccordements à électrode et est conçue pour exploiter des potentiels électriques appliqués au niveau des raccordements à électrode, notamment à capter au niveau intracardiaque, afin de détecter les phénomènes atriaux intrinsèques caractéristiques des contractions naturelles d'un atrium,
   l'unité de commande de stimulation étant conçue pour,
   aux fins du contrôle de réussite de stimulation (détection de capture) dans l'atrium, déclencher au moins une impulsion de stimulation atriale et détecter une stimulation réussie (capture) dans l'atrium ou une stimulation atriale inefficace (non-capture) en partant de la non-détection ou de la détection d'au moins un phénomène atrial intrinsèque (naturel, spontané) survenant le cas échéant pendant une durée prédéfinie,
   **caractérisé en ce que** l'unité de commande de stimulation est en outre conçue pour
   déclencher au moins des impulsions de stimulation ventriculaires utiles pour une période limitée prédéfinie servant au contrôle de réussite de stimulation (détection de capture) ou à un test de seuil d'excitation et incluant plusieurs cycles cardiaques à partir du début d'un intervalle AV commençant par un phénomène atrial intrinsèque ou stimulé et qui est assez court pour éviter des phénomènes de fusion, c'est-à-dire des phénomènes lors desquels une impulsion de stimulation ventriculaire a lieu sensiblement en même temps qu'une contraction ventriculaire naturelle, et

à déclencher, sur la période prédéfinie, des impulsions de stimulation à un rythme de stimulation réglé et/ou en mode déclenché de manière à ne pas inhiber les impulsions de stimulation, et

à distinguer les phénomènes atriaux qui reposent sur la perception de champ lointain des phénomènes ventriculaires dans l'atrium de phénomènes atriaux intrinsèques du fait que ce type de phénomènes atriaux qui ont lieu avec un intervalle temporel par rapport à l'impulsion de stimulation ventriculaire respectivement précédente et qui équivaut, dans d'étroites limites de tolérance, à l'intervalle temporel entre un autre phénomène atrial et l'impulsion de stimulation ventriculaire respectivement précédente sont évalués en se basant sur la perception de champ lointain et ne sont pas décomptés comme phénomènes intrinsèques, et

à détecter les phénomènes atriaux intrinsèques (naturels, spontanés) survenant le cas échéant pour la période prédéfinie, et

à déterminer pour la période prédéfinie, en se basant sur les phénomènes atriaux intrinsèques (naturels, spontanés) détectés ou non détectés, la présence ou la non-présence d'un rythme cardiaque naturel.

2. Stimulateur électrique selon la revendication 1, **caractérisé en ce que** l'unité de commande de stimulation est conçue pour générer un signal de champ lointain caractéristique d'un phénomène atrial reposant sur une perception de champ lointain lorsque le phénomène atrial détecté par l'unité de détection tombe dans un intervalle prédéfini de couplage de champ lointain qui démarre à partir d'une impulsion de stimulation ventriculaire respective et se termine au plus tard 150 ms après le déclenchement d'une impulsion de stimulation ventriculaire.

3. Stimulateur électrique selon la revendication 2, **caractérisé en ce que** l'intervalle de couplage de champ lointain démarre 50 à 100 ms après le déclenchement de l'impulsion de stimulation ventriculaire et se termine 150 à 200 ms après le déclenchement de la même impulsion de stimulation ventriculaire.

4. Stimulateur électrique selon une des revendications 1 à 3, **caractérisé en ce que** l'unité de commande de stimulation est conçue pour régler la durée AV pendant la période prédéfinie de manière à ce que la durée AV soit assez courte pour qu'il n'y ait pas de battements de fusion dans le ventricule et qu'il n'y ait pas de conductions rétrogrades du ventricule à l'atrium lors d'une stimulation atriale et ventriculaire effective.

5. Stimulateur électrique selon la revendication 4, **caractérisé en ce que** l'intervalle AV dure moins de 200 ms et a de préférence une durée allant de 100 à 130 ms.

6. Stimulateur électrique selon une des revendications 1 à 5, **caractérisé en ce que** l'unité de commande de stimulation est conçue pour exploiter également les phénomènes ayant lieu pendant une période d'extinction après la stimulation ventriculaire de manière à ce que tous les phénomènes survenant pendant la période d'extinction soient ensuite catégorisés comme phénomènes intrinsèques pour lesquels il n'existe pendant la période prédéfinie aucun autre phénomène atrial qui suit ou a suivi une impulsion de stimulation ventriculaire respectivement précédente avec un intervalle de couplage identique ou similaire.

7. Stimulateur électrique selon une des revendications 1 à 6, **caractérisé en ce que** l'unité de commande de stimulation est conçue pour générer un signal conducteur d'excitation rétrograde dans le cas où l'unité de détection détecte un phénomène atrial intrinsèque pendant un intervalle temporel prédéfini après déclenchement d'une impulsion de stimulation ventriculaire.

8. Stimulateur électrique selon la revendication 7, **caractérisé en ce que** l'intervalle temporel prédéfini est un intervalle de couplage de 180 ms à 350 ms entre le déclenchement de l'impulsion de stimulation ventriculaire et la détection du phénomène atrial intrinsèque.

9. Stimulateur électrique selon la revendication 7 ou 8, **caractérisé en ce que** l'unité de commande de stimulation est conçue pour générer un signal de perte de capture (LOC) dans le cas où le rapport entre le nombre de signaux conducteurs d'excitation rétrograde et le nombre d'impulsions de stimulation ventriculaire déclenchées dépasse pendant une période prédéfinie une valeur seuil prédéfinie comprise entre 0,5 et 1.

10. Stimulateur électrique selon la revendication 9, **caractérisé en ce que** la valeur seuil prédéfinie s'élève à 0,8.

11. Stimulateur électrique selon la revendication 1, **caractérisé en ce que** l'unité de commande de stimulation est conçue pour générer un signal de diaphonie dans le cas où l'unité de détection détecte un phénomène atrial intrinsèque pendant une fenêtre de temps prédéfinie qui commence au déclenchement d'une impulsion de stimulation ventriculaire respective.

**12.** Stimulateur électrique selon la revendication 11, **caractérisé en ce que** la fenêtre de temps prédéfinie a une durée de 30 ms à 70 ms, de préférence 70 ms.

**13.** Stimulateur électrique selon la revendication 11 ou 12, **caractérisé en ce que** l'unité de détection est conçue pour détecter seulement les phénomènes atriaux en tant que phénomènes intrinsèques auxquels aucun signal de diaphonie ou signal de champ lointain ne doit être affecté.

**14.** Stimulateur électrique selon une des revendications 1 à 13, **caractérisé en ce que** l'unité de commande de stimulation est conçue pour inhiber le déclenchement d'une impulsion de stimulation ventriculaire dans le cas où, pendant l'intervalle AV, aucun phénomène ventriculaire intrinsèque n'est détecté.

**15.** Stimulateur électrique selon la revendication 1, **caractérisé en ce que** l'unité de commande de stimulation est conçue pour dériver le rythme de stimulation réglé d'un rythme existant avant la période prédéfinie incluant plusieurs cycles cardiaques de manière à ce que le rythme de stimulation réglé soit supérieur au rythme précédemment existant.

**16.** Stimulateur électrique selon la revendication 1 ou 15, **caractérisé en ce que** l'unité de commande de stimulation est conçue pour régler dans un mode inhibé le rythme de stimulation auquel l'émission d'impulsions de stimulation n'est pas inhibée par augmentation répétée du rythme de stimulation à raison d'une valeur prédéfinie jusqu'à ce qu'il n'y ait plus d'inhibition.

**17.** Stimulateur électrique selon la revendication 1 ou 5, **caractérisé en ce que** l'unité de commande de stimulation est conçue pour définir le rythme de stimulation auquel l'émission d'impulsions de stimulation n'est pas inhibée dans un mode déclenché par augmentation en une fois du rythme de stimulation à raison d'une valeur prédéfinie.

**18.** Stimulateur électrique selon une des revendications 1 ou 17, **caractérisé en ce que** le stimulateur électrique sous forme de stimulateur du rythme cardiaque, l'unité de stimulation destinée à générer et à émettre des impulsions de stimulation atriales, l'unité de détection destinée à détecter des phénomènes atriaux intrinsèques et l'unité de commande de stimulation destinée à déterminer un rythme cardiaque atrial naturel sont réalisés à partir de phénomènes intrinsèques détectés par l'unité de détection dans l'atrium.

**19.** Stimulateur électrique selon la revendication 18, **caractérisé en ce que** l'unité de commande de stimulation est conçue pour déclencher, dans la période prédéfinie incluant plusieurs cycles cardiaques,
des impulsions de stimulation atriales à un rythme de stimulation réglé dans un mode déclenché ou à une vitesse de stimulation atriale réglée de manière à ce que les impulsions de stimulation ventriculaires ne soient pas inhibées et des impulsions de stimulation ventriculaires à un rythme de stimulation réglé dans un mode demande dans lequel les impulsions de stimulation ventriculaires sont inhibées dans le cas où, pendant un intervalle d'échappement, des phénomènes ventriculaires intrinsèques sont détectés.

**20.** Stimulateur électrique selon une des revendications 1 à 19, **caractérisé en ce que** l'unité de commande de stimulation est conçue pour déterminer la présence ou la non-présence d'un rythme cardiaque naturel selon que et et/ou selon le nombre de phénomènes intrinsèques détectés par l'unité de détection pendant la période prédéfinie.

**21.** Stimulateur électrique selon une des revendications 1 à 20, **caractérisé en ce que** l'unité de commande de stimulation est conçue pour tenir compte, pour la détermination de la présence ou la non-présence d'un rythme cardiaque naturel, de temps de disparition (durées d'extinction) au cours desquels les phénomènes naturels survenant éventuellement ne peuvent pas être détectés.

**22.** Stimulateur électrique selon la revendication 21, **caractérisé en ce que** l'unité de commande de stimulation est conçue pour rétablir un rythme cardiaque naturel à partir de phénomènes naturels détectés en tenant compte de phénomènes naturels potentiellement survenus dans les temps de disparition et générer un signal caractéristique d'un rythme cardiaque naturel dans le cas où les phénomènes naturels détectés s'associent à un rythme cardiaque naturel régulier.

**23.** Stimulateur électrique selon une des revendications 1 à 22, **caractérisé en ce que** l'unité de détection est conçue pour générer, pour chaque phénomène intrinsèque détecté, un signal marqueur de détection en association chronologique relative par rapport au moment de la survenance du phénomène.

24. Stimulateur électrique selon la revendication 23, **caractérisé en ce que** l'unité de commande de stimulation est conçue pour générer respectivement un signal marqueur de rythme pour chaque impulsion de stimulation déclenchée et déterminer la présence d'un rythme cardiaque naturel au moins à partir des signaux marqueurs de détection ou des signaux marqueurs de rythme en supplément.

25. Stimulateur électrique selon une des revendications 1 à 24, **caractérisé en ce que** l'unité de commande de stimulation est conçue pour définir un signal de commande de stimulation pour l'intensité de stimulation selon qu'une présence ou une non-présence d'un rythme naturel est déterminée pendant la période prédéfinie.

26. Stimulateur électrique selon la revendication 25, **caractérisé en ce que** l'unité de commande de stimulation est conçue pour générer un signal de commande de stimulation qui provoque un accroissement de l'intensité d'impulsion d'une impulsion de stimulation lorsqu'une présence d'un rythme cardiaque naturel est déterminée pendant la durée prédéfinie.

27. Stimulateur électrique selon une des revendications 1 à 26, **caractérisé en ce que** l'unité de commande de stimulation est conçue pour définir un signal de commande de capture selon qu'une présence ou une non-présence d'un rythme cardiaque naturel est déterminée pendant la durée prédéfinie et, en complément ou en variante, pour générer pendant la durée prédéfinie un signal de perte de capture (LOC) dans le cas de la présence d'un rythme cardiaque naturel.

28. Stimulateur électrique selon la revendication 1, **caractérisé en ce que** l'unité de commande de stimulation est conçue pour émettre plusieurs impulsions de stimulation au rythme de stimulation dérivé accru et générer un signal de commande de stimulation qui provoque un accroissement de l'intensité d'impulsion d'une impulsion de stimulation lorsque, pendant la durée prédéfinie, le rapport entre le nombre d'impulsions émises au rythme de stimulation dérivé accru et le nombre de phénomènes intrinsèques détectés pendant la période prédéfinie dépasse une valeur prédéfinie.

FIG. 1

EP 1 582 234 B1

V TRIG | V-STIM AMPL

A TRIG | A-STIM AMPL

V - STIM — 30

A-STIM — 32

CAP. DETEC. — 52

V-SENSE — 44

A-SENSE — 42

10

50

34

36

38

40

12

14

16

18

20

24

30

FIG. 2

FIG. 3

RA-ATT Capture Detection Algorithm
(see ACC_Annnotation_v4.m)

**FIG. 4**

FIG. 5a

FIG. 5b

FIG. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5601615 A
- US 5861012 A
- US 6584354 B
- US 20040030358 A